# EUROPEAN PATENT SPECIFICATION

(11) **EP 2 598 518 B1**
(45) Date of publication and mention of the grant of the patent: **30.01.2019**
(21) Application number: 10754989.1
(22) Date of filing: 28.07.2010
(51) Int. Cl.: C07K 7/04, C07K 14/78, C12N 5/00, C12N 5/07

(54) **PRE-POLYMER PREPARATIONS FOR CELL CULTURE COATINGS**
PRÄPOLYMERZUBEREITUNGEN FÜR ZELLKULTURBESCHICHTUNGEN
PRÉPARATIONS PRÉ-POLYMÈRES POUR DES REVÊTEMENTS DE CULTURE CELLULAIRE

(43) Date of publication of application: 05.06.2013
(73) Proprietor: Corning Incorporated, Corning, New York 14831 (US)
(72) Inventor: HENRY, David, F-91150 Morigny-Champigny (FR); HERVY, Martial, F-77810 Thomery (FR); MARTIN, Arthur, Winston, Horseheads New York 14845 (US); BOOKBINDER, Dana, Craig, Corning New York 14830 (US); O'MALLEY, Shawn, Michael, Horseheads New York 14845 (US)
(74) Representative: Hart-Davis, Jason
(86) International application number: PCT/IB2010/002160
(87) International publication number: WO 2012/014003

(56) References cited:
- WO-A1-2011/103961
- WO-A2-2006/105278
- WO-A2-2009/099555
- US-A1- 2004 209 361
- US-A1- 2005 282 747
- US-A1- 2007 167 354
- HERN D L ET AL: "INCORPORATION OF ADHESION PEPTIDES INTO NONADHESIVE HYDROGELS USEFUL FOR TISSUE RESURFACING", JOURNAL OF BIOMEDICAL MATERIALS RESEARCH, WILEY, NEW YORK, NY, US, vol. 39, 1 January 1998 (1998-01-01), pages 266-276, XP002949005, ISSN: 0021-9304, DOI: DOI:10.1002/(SICI)1097-4636(199802)39:2<26 6::AID-JBM14>3.0.CO;2-B
- DAWSON ET AL: "Biomaterials for stem cell differentiation", ADVANCED DRUG DELIVERY REVIEWS, ELSEVIER BV, AMSTERDAM, NL, vol. 60, no. 2, 11 October 2007 (2007-10-11), pages 215-228, XP022388009, ISSN: 0169-409X, DOI: DOI:10.1016/J.ADDR.2007.08.037
- MELKOUMIAN ZARA ET AL: "Synthetic peptide-acrylate surfaces for long-term self-renewal and cardiomyocyte differentiation of human embryonic stem cells", NATURE BIOTECHNOLOGY, NATURE PUBLISHING GROUP, NEW YORK, NY, US, vol. 28, no. 6, 1 June 2010 (2010-06-01), pages 606-610, XP002636474, ISSN: 1087-0156, DOI: DOI:10.1038/NBT.1629 [retrieved on 2010-05-30]
- LI Y ET AL: "Expansion of human embryonic stem cells in defined serum-free medium devoid of animal-derived products", BIOTECHNOLOGY AND BIOENGINEERING, WILEY & SONS, HOBOKEN, NJ, US, vol. 91, no. 6, 20 September 2005 (2005-09-20), pages 688-698, XP002351170, ISSN: 0006-3592, DOI: DOI:10.1002/BIT.20536
- LI Y J ET AL: "Hydrogels as artificial matrices for human embryonic stem cell self-renewal", JOURNAL OF BIOMEDICAL MATERIALS RESEARCH. PART A, WILEY PERIODICALS INC, HOBOKEN, NY, US, vol. 79a, no. 1, 1 October 2006 (2006-10-01), pages 1-5, XP008114847, ISSN: 1549-3296, DOI: 10.1002/JBM.A.30732 [retrieved on 2006-06-01]

### SEQUENCE LISTING

<110> CORNING INCORPORATED
<120> PRE-POLYMER PREPARATIONS FOR CELL CULTURE COATINGS
<130> SP10-205
<160> 33
<170> PatentIn version 3.5
<210> 1
   <211> 20
   <212> PRT
   <213> SYNTHETIC PEPTIDE
<400> 1 Cys Ser Lys Ser 20
<210> 2
   <211> 19
   <212> PRT
   <213> SYNTHETIC PEPTIDE
<400> 2 Ser Lys Ser
<210> 3
   <211> 13
   <212> PRT
   <213> SYNTHETIC PEPTIDE
<400> 3
   Lys Tyr Gly Leu Ala Leu Glu Arg Lys Asp His Ser Gly
   1 5 10
<210> 4
   <211> 12
   <212> PRT
   <213> SYNTHETIC PEPTIDE
<400> 4
   Tyr Gly Leu Ala Leu Glu Arg Lys Asp His Ser Gly
   1 5 10
<210> 5
   <211> 23
   <212> PRT
   <213> SYNTHETIC PEPTIDE
<400> 5 Arg Phe Gly Asn Met Gly Ser 20
<210> 6
   <211> 22
   <212> PRT
   <213> SYNTHETIC PEPTIDE
<400> 6 Phe Gly Asn Met Gly Ser 20
<210> 7
   <211> 15
   <212> PRT
   <213> SYNTHETIC PEPTIDE
<400> 7
   Lys Gly Gly Thr Trp Tyr Lys Ile Ala Phe Gln Arg Asn Arg Lys
   1 5 10 15
<210> 8
   <211> 14
   <212> PRT
   <213> SYNTHETIC PEPTIDE
<400> 8
   Gly Gly Thr Trp Tyr Lys Ile Ala Phe Gln Arg Asn Arg Lys
   1 5 10
<210> 9
   <211> 15
   <212> PRT
   <213> SYNTHETIC PEPTIDE
<400> 9
   Lys Gly Gly Thr Ser Ile Lys Ile Arg Gly Thr Tyr Ser Glu Arg
   1 5 10 15
<210> 10
   <211> 14
   <212> PRT
   <213> SYNTHETIC PEPTIDE
<400> 10
   Gly Gly Thr Ser Ile Lys Ile Arg Gly Thr Tyr Ser Glu Arg
   1 5 10
<210> 11
   <211> 16
   <212> PRT
   <213> SYNTHETIC PEPTIDE
<400> 11
   Lys Tyr Gly Thr Asp Ile Arg Val Thr Leu Asn Arg Leu Asn Thr Phe
   1 5 10 15
<210> 12
   <211> 15
   <212> PRT
   <213> SYNTHETIC PEPTIDE
<400> 12
   Tyr Gly Thr Asp Ile Arg Val Thr Leu Asn Arg Leu Asn Thr Phe
   1 5 10 15
<210> 13
   <211> 16
   <212> PRT
   <213> SYNTHETIC PEPTIDE
<400> 13
   Lys Tyr Gly Ser Glu Thr Thr Val Lys Tyr Ile Phe Arg Leu His Glu
   1 5 10 15
<210> 14
   <211> 15
   <212> PRT
   <213> SYNTHETIC PEPTIDE
<400> 14
   Tyr Gly Ser Glu Thr Thr Val Lys Tyr Ile Phe Arg Leu His Glu
   1 5 10 15
<210> 15
   <211> 15
   <212> PRT
   <213> SYNTHETIC PEPTIDE
<400> 15
   Lys Tyr Gly Lys Ala Phe Asp Ile Thr Tyr Val Arg Leu Lys Phe
   1 5 10 15
<210> 16
   <211> 14
   <212> PRT
   <213> SYNTHETIC PEPTIDE
<400> 16
   Tyr Gly Lys Ala Phe Asp Ile Thr Tyr Val Arg Leu Lys Phe
   1 5 10
<210> 17
   <211> 15
   <212> PRT
   <213> SYNTHETIC PEPTIDE
<400> 17
   Lys Tyr Gly Ala Ala Ser Ile Lys Val Ala Val Ser Ala Asp Arg
   1 5 10 15
<210> 18
   <211> 14
   <212> PRT
   <213> SYNTHETIC PEPTIDE
<400> 18
   Tyr Gly Ala Ala Ser Ile Lys Val Ala Val Ser Ala Asp Arg
   1 5 10
<210> 19
   <211> 15
   <212> PRT
   <213> SYNTHETIC PEPTIDE
<400> 19
   Cys Gly Gly Asn Gly Glu Pro Arg Gly Asp Thr Tyr Arg Ala Tyr
   1 5 10 15
<210> 20
   <211> 14
   <212> PRT
   <213> SYNTHETIC PEPTIDE
<400> 20
   Gly Gly Asn Gly Glu Pro Arg Gly Asp Thr Tyr Arg Ala Tyr
   1 5 10
<210> 21
   <211> 14
   <212> PRT
   <213> SYNTHETIC PEPTIDE
<400> 21
   Cys Gly Gly Asn Gly Glu Pro Arg Gly Asp Thr Arg Ala Tyr
   1 5 10
<210> 22
   <211> 13
   <212> PRT
   <213> SYNTHETIC PEPTIDE
<400> 22
   Gly Gly Asn Gly Glu Pro Arg Gly Asp Thr Arg Ala Tyr
   1 5 10
<210> 23
   <211> 15
   <212> PRT
   <213> SYNTHETIC PEPTIDE
<400> 23
   Lys Tyr Gly Arg Lys Arg Leu Gln Val Gln Leu Ser Ile Arg Thr
   1 5 10 15
<210> 24
   <211> 14
   <212> PRT
   <213> SYNTHETIC PEPTIDE
<400> 24
   Tyr Gly Arg Lys Arg Leu Gln Val Gln Leu Ser Ile Arg Thr
   1 5 10
<210> 25
   <211> 13
   <212> PRT
   <213> SYNTHETIC PEPTIDE
<400> 25
   Lys Gly Gly Arg Asn Ile Ala Glu Ile Ile Lys Asp Ile
   1 5 10
<210> 26
   <211> 12
   <212> PRT
   <213> SYNTHETIC PEPTIDE
<400> 26
   Gly Gly Arg Asn Ile Ala Glu Ile Ile Lys Asp Ile
   1 5 10
<210> 27
   <211> 15
   <212> PRT
   <213> SYNTHETIC PEPTIDE
<400> 27
   Lys Gly Gly Pro Gln Val Thr Arg Gly Asp Val Phe Thr Met Pro
   1 5 10 15
<210> 28
   <211> 16
   <212> PRT
   <213> SYNTHETIC PEPTIDE
<400> 28
   Ser Tyr Asn Thr His Glu Thr Ile Cys Pro Glu Pro Thr Ile Asp Glu
   1 5 10 15
<210> 29
   <211> 7
   <212> PRT
   <213> SYNTHETIC PEPTIDE
<400> 29
   Gly Arg Gly Asp Ser Pro Lys
   1 5
<210> 30
   <211> 15
   <212> PRT
   <213> SYNTHETIC PEPTIDE
<400> 30
   Lys Gly Gly Ala Val Thr Gly Arg Gly Asp Ser Pro Ala Ser Ser
   1 5 10 15
<210> 31
   <211> 14
   <212> PRT
   <213> SYNTHETIC PEPTIDE
<400> 31
   Gly Gly Ala Val Thr Gly Arg Gly Asp Ser Pro Ala Ser Ser
   1 5 10
<210> 32
   <211> 14
   <212> PRT
   <213> SYNTHETIC PEPTIDE
<220>
   <221> misc_feature
   <222> (1)..(1)
   <223> Xaa can be any naturally occurring amino acid
<400> 32
   Xaa Leu Pro Gln Val Thr Arg Gly Asn Val Phe Thr Met Pro
   1               5                   10
<210> 33
   <211> 5
   <212> PRT
   <213> SYNTHETIC PEPTIDE
<400> 33
   Arg Gly Asp Tyr Lys
   1               5

## Description

### FIELD

The present disclosure relates to methods of making synthetic cell culture articles. More particularly, the present disclosure relates to methods of making cell culture articles by cross-linking functionalized cell binding peptide pre-polymers with cross-linker-functionalized pre-polymers to form cross-linked polymer blends suitable as cell culture surfaces. In addition, the disclosure relates to synthetic surfaces and articles for supporting the culture of cells in chemically defined medium.

### SEQUENCE LISTING

This application contains a Sequence Listing. The information contained in the Sequence Listing is hereby incorporated herein by reference.

### BACKGROUND

Therapeutic cells, cells which may be introduced into a human for the treatment of disease, are being developed. Examples of therapeutic cells include pluripotent stem cells such as human embryonic stem cells (hESCs) which have the ability to differentiate into any of the three germ layers, giving rise to any adult cell type in human body. This property of stem cells provides a potential for developing new treatments for a number of serious cell degenerative diseases, such as diabetes, spinal chord injury, heart diseases and the like. However there remain obstacles in the development of such hESC-based treatments. Obtaining and maintaining adequate numbers of therapeutic cells in cell and tissue culture and ensuring that these cells do not change in unwanted ways during cell culture are important in developing and controlling therapeutic cell cultures. For example, stem cell cultures, such as hESC

cell cultures are typically seeded with a small number of cells from a cell bank or stock and then amplified in the undifferentiated state until differentiation is desired for a given therapeutic application. To accomplish this, the hESC or their differentiated cells are currently cultured in the presence of surfaces or media containing animal-derived components, such as feeder layers, serum, or Matrigel™ available from BD Biosciences, Franklin Lakes NJ. These animal-derived additions to the culture environment expose the cells to potentially harmful viruses or other infectious agents which could be transferred to patients or compromise general culture and maintenance of the hESCs. In addition, such biological products are vulnerable to batch variation, immune response and limited shelf-life. Therefore, methods of producing synthetic cell culture surfaces which are capable of supporting cells, including therapeutic cells in culture, in chemically defined or serum-free media, and the surfaces so produced, are desirable.

### SUMMARY

In aspect (1), corresponding to the invention as defined in the appended claims, a composition is provided for forming a polymeric cell culture surface comprising: a first pre-polymer wherein the first pre-polymer comprises a cell adhesive peptide conjugated to a first polymer backbone; and, a second pre-polymer wherein the second pre-polymer comprises a cross-linker moiety conjugated to a second polymer backbone, wherein the second pre-polymer comprises the polymerization product of an ethylenically unsaturated monomer and the cross-linker moiety is a photo-reactive group selected from the group consisting of aryl azides, diazarenes, beta carbonyldiazo, benzophenones, and acetophenones. In an aspect (2), the composition of aspect 1 is provided wherein the cell adhesive peptide comprises an R-G-D sequence. In an aspect (3) the composition of aspect 1 or 2 is provided wherein the cell adhesive peptide is a sequence of amino acids found in vitronectin, laminin, bone sialoprotein, collagen, or fibronectin. In an aspect (4), the composition of any one of aspects 1-4 is provided wherein the cell adhesive peptide is a sequence of amino acids selected from the group consisting of: KGGGQKCIVQTTSWSQCSKS (SEQ ID NO:1), GGGQKCIVQTTSWSQCSKS (SEQ ID NO:2), KYGLALERKDHSG (SEQ ID NO:3), YGLALERKDHSG (SEQ ID NO:4), KGGSINNNRWHSIYITRFGNMGS (SEQ ID NO:5), GSINNNRWHSIYITRFGNMGS (SEQ ID NO:6), KGGTWYKIAFQRNRK (SEQ ID NO:7), GGTWYKIAFQRNRK (SEQ ID NO:8), KGGTSIKIRGTYSER (SEQ ID NO:9), GGTSIKIRGTYSER (SEQ ID NO:10),
KYGTDIRVTLNRLNTF (SEQ ID NO:11), YGTDIRVTLNRLNTF (SEQ ID NO:12), KYGSETTVKYIFRLHE (SEQ ID NO:13), YGSETTVKYIFRLHE (SEQ ID NO:14), KYGKAFDITYVRLKF (SEQ ID NO:15), YGKAFDITYVRLKF (SEQ ID NO:16), KYGAASIKVAVSADR (SEQ ID NO:17), YGAASIKVAVSADR (SEQ ID NO:18), CGGNGEPRGDTYRAY (SEQ ID NO:19), GGNGEPRGDTYRAY (SEQ ID NO:20), CGGNGEPRGDTRAY (SEQ ID NO:21), GGNGEPRGDTRAY (SEQ ID NO:22), KYGRKRLQVQLSIRT (SEQ ID NO:23), YGRKRLQVQLSIRT (SEQ ID NO:24), KGGRNIAEIIKDI (SEQ ID NO:25), GGRNIAEIIKDI (SEQ ID NO:26), KGGPQVTRGDVFTMP (SEQ ID NO:27), GGPQVTRGDVFTMP (SEQ ID NO:28), GRGDSPK (SEQ ID NO:29), KGGAVTGRGDSPASS (SEQ ID NO:30), GGAVTGRGDSPASS (SEQ ID NO:31), Yaa₁PQVTRGNVFTMP (SEQ ID NO:32), RGDYK (SEQ ID NO:33), and combinations. In an aspect (5), the composition of any one of aspects 1-4 is provided wherein the first pre-polymer comprising the polymerization product of a functionalized cell adhesive peptide of the formula Rₘ - Sₚ - Cₐₚ; wherein R is a polymerization moiety selected from the group consisting of acrylate, methacrylate, acrylamide, methacrylamide, maleimide and fumarate. and combinations, and m is an integer greater than 1; wherein Sₚ is an optional spacer moiety wherein the spacer moiety comprises polyethylene oxide or polypropylene oxide having the formula (O-CH₂CHR')ₘ₂ where R' is H or CH₃ and m2 is an integer from 0 to 20, or Xaaₙ wherein Xaa is independently any amino acid and n is an integer from 0 to 3, or a combination; and, wherein Cₐₚ is a peptide comprising a cell adhesive sequence. In an aspect (6), the composition of any one of aspects 1-5 is provided wherein the first pre-polymer comprises the polymerization product of an ethylenically unsaturated monomer having a carboxyl group with an ethylenically unsaturated monomer not having a carboxyl group, wherein the cell adhesive peptide is conjugated to the first pre-polymer via the carboxyl group. In an aspect (7), the composition of any one of aspects 1-6 is provided wherein the ethylenically unsaturated monomer of the first pre-polymer is hydrophilic. In an aspect (8), the composition of any one of aspects 1-7 is provided wherein the ethylenically unsaturated monomer of the first pre-polymer not having a carboxyl group is hydrophilic.

In an aspect (9), the composition of aspect 1 is provided wherein the ethylenically unsaturated monomer of the second pre-polymer is hydrophilic. In an aspect (10), the composition of aspect 9 is provided wherein the second pre-polymer comprises a benzophenone cross-linker moiety conjugated to poly-HEMA.. In an aspect (11), the composition of any one of aspects 5, 6, 9 and 10 wherein the ethylenically unsaturated monomer which does not have a carboxyl group comprising the first pre-polymer and the ethylenically unsaturated monomer comprising the second pre-polymer are the same.

In an additional aspect, a method of making a cell culture article comprising providing the first and second pre-polymers of any one of aspects 1 to 11 to a substrate and exposing the first and second pre-polymers on the substrate to an energy source to cross-link the first and second pre-polymer to each other and to the substrate is provided. In an aspect (13), a cell culture article made by the method of aspect 12 is provided.

### BRIEF DESCRIPTION OF THE DRAWINGS

**FIG. 1** is an illustration showing a method for making Poly(HEMA co-MAA-PEG₄-VN), an embodiment of the first pre-polymer, containing a cell adhesive peptide.
**FIG. 2** is diagram illustrating a reaction for forming an alternative embodiment of the first pre-polymer, by first providing HEMA-co-CEA, and then conjugating a peptide to form HEMA-co-CEA-peptide.
**FIG. 3** is an illustration showing a method for making an embodiment of a second pre-polymer, a Photo-HEMA pre-polymer.
**FIG. 4** is an illustration of a combination of pre-polymers Photo-HEMA and Poly(HEMA co-MAA-PEG₄-VN) for making an embodiment of a cell culture surface.
**FIG. 5** is another illustration showing cross-linking of pre-polymers Photo-HEMA and Poly(HEMA co-MAA-PEG₄-VN) to form embodiment of a cell culture surface.
**FIG. 6** is an illustration of another embodiment of a cross-linked pre-polymeric cell culture surfaces, combining Photo-HEMA and Poly(HEMA co-CEA-VN).
**FIG. 7** is a graph showing the amount of immobilized cell culture coating as function of the percent of the second pre-polymer (the photoreactive pre-polymer) forming copolymer in the two components mixture.
**FIG. 8** is a graph illustrating the amount of immobilized hydrogel as function of the concentration of the first pre-polymer, the peptide-containing pre-polymer in (mg/ml) in the solution used for coating.
**FIG. 9** is a graph illustrating the amount of immobilized peptide (pmol/mm²) as function of the percentage by weight of the second pre-polymer, the peptide-conjugated pre-polymer, in the two components mixture.
**FIG. 10** is a photograph showing colloidal gold staining of a photohydrogel-coated flask according to an embodiment and an uncoated control flask.
**FIG. 11A** and **B** are photomicrographs illustrating HT1080 cells on an embodiment of the cell culture surface of the present invention (A) and on a control surface (B) without cell adhesive peptide.
**FIG. 12A-D** are photomicrographs showing Nulli-SCCl cells on Synthemax™ from Corning, Incorporated (FIG. 12A), on VN-HEMA-CEA (FIG. 12B) and on HEMA-CEA (without cell adhesive peptide) (FIG. 12C). FIG. 12D is a graph illustrating OD measurements of cell growth on the three surfaces illustrated in FIGs. 12A-C.
**FIG. 13A-D** are photomicrographs showing ES-D3 cells on Synthemax™ from Corning, Incorporated (FIG. 13A), on VN-HEMA-CEA (FIG. 13B) and on HEMA-CEA (without cell adhesive peptide) (FIG. 13C). FIG. 13D is a graph illustrating OD measurements of cell growth on the three surfaces illustrated in FIGs. 13A-C.
**FIG. 14A-D** are photomicrographs showing ES-D3 mouse embryonic stem cell adhesion and morphology after 48 hours on Matrigel® available from BD, Franklin Lakes, NJ (FIG. 14A), Synthemax ™ available from Corning Incorporated, Corning, NY (FIG. 14B) embodiments of the polymer coating Poly(HEMA co-VN-PEG₄-MAA - 10%) (FIG. 14C) and a negative control of PhotoHEMA + peptide (FIG. 14D).

### DETAILED DESCRIPTION

The present invention provides a composition for forming a synthetic polymeric cell culture surface comprising: a first pre-polymer wherein the first pre-polymer comprises a cell adhesive peptide conjugated to a first polymer backbone and a second pre-polymer wherein the second pre-polymer comprises a cross-linker moiety conjugated to a second polymer backbone wherein the second pre-polymer comprises the polymerization product of an ethylenically unsaturated monomer and the cross-linker moiety is a photo-reactive group selected from the group consisting of aryl azides, diazarenes, beta carbonyldiazo, benzophenones, and acetophenones. In embodiments, the disclosure also provides methods of making a cell culture surface comprising providing the first pre-polymer with the second pre-polymer, in the presence of cell culture substrate or base, exposing the first pre-polymer and second pre-polymer mixture to an energy source, to form a reactive polymer blend conjugated to the cell culture base. In additional embodiments, the disclosure provides synthetic cell culture surfaces made from the pre-polymer compositions and methods for culturing cells on the synthetic cell culture surfaces so made.

In the following detailed description, reference is made to the accompanying drawings that form a part hereof, and in which are shown by way of illustration several specific embodiments of devices, systems and methods.

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise. As used in this specification and the appended claims, the term "or" is generally employed in its sense including "and/or" unless the content clearly dictates otherwise.

As used herein, "have", "having", "include", "including", "comprise", "comprising" or the like are used in their open ended sense, and generally mean "including, but not limited to".

Synthetic cell culture surfaces including surfaces that incorporate synthetic or recombinant proteins or peptides, for example, are suitable for supporting cells that may be introduced into a human for the treatment of disease. Synthetic peptides and proteins may include cell adhesive sequences such as RGD. Polypeptide sequences are referred to herein by their one letter amino acid codes and by their three letter amino acid codes. These codes may be used interchangeably. Synthetic surfaces that reduce the amount of peptide required to support viable cells in culture are desirable, because peptides can expensive, and so surfaces requiring less peptide are less expensive. In addition, synthetic surfaces that are easy to manufacture, stable in storage, stable through sterilization procedures, and stable through long exposure to aqueous cell culture conditions are also desirable.

In embodiments of the present invention, a first pre-polymer is provided wherein the first pre-polymer comprises a cell adhesive peptide conjugated to a first polymer backbone.

In embodiments, the first pre-polymer is formed from monomers which have peptides or polypeptides which have been modified or functionalized to carry a polymerization moiety with an ethylenically unsaturated monomer such as an acrylate, methacrylate, acrylamide, methacrylamide, maleimide or fumarate are polymerized to form a first pre-polymer. For the purposes of this disclosure "functionalized peptide" means peptides which have been modified to incorporate polymerization moieties such as acrylate, methacrylate, acrylamide, methacryalmide, maleimide or fumarate groups. In embodiments these polymerization moieties can form polymers in the presence of an external energy source such as UV or visible light with an optional catalyst, or by thermal polymerization with an optional catalyst. In embodiments, the functionalized peptides or polypeptides may contain a spacer moiety.

In embodiments, the functionalized peptide is described by formula 1:

### Formula 1: Rₘ - Sₚ - Cₐₚ

In embodiments, Rₘ is a polymerization moiety, an α, β-unsaturated group or ethylenically unsaturated group which includes acrylate, methacrylate, acrylamide, methyacrylamide, maleimide or a fumarate, which is capable of polymerizing in the presence of an external energy source. "m" is an integer greater than or equal to 1. In embodiments, the functionalized peptide has a polymerization moiety Rₘ which may be a photopolymerizable moiety or a thermal polymerizable moiety.

In embodiments, Sₚ is a spacer. In embodiments, Sₚ may be a polyalkylene oxide including for example polyethylene glycol (PEG) or polypropylene glycol (PPG) which are represented by the formula (O-CH₂CHR')ₘ₂ where R' is H or CH₃ and m2 is an integer from 0 to 20. The spacer may be a hydrophilic spacer, for example, polyethelene oxide (PEO). In embodiments, the spacer is PEO₄. In embodiments, relatively short chains of polyalkylene oxide are desirable. For example, in embodiments, Sₚ may be PEG₂, PEG₄, PEG₆, PEG₈, PEG₁₀, PEG₁₂ or PPG₂, PPG₄, PPG₆, PPG₈, PPG₁₀, PPG₁₂ or PPG₂₀. In embodiments, the spacer is a polyethylene oxide with 20 or fewer repeating units (i.e. PEG₄, PEG₆, PEG₈, PEG₁₀, PEG₁₂, PEG₁₄, PEG₁₆, PEG₁₈ or PEG₂₀). In embodiments Sₚ is PPG or PEG having a functional group. For example, the PEG or PPG spacer may have a maleimide, thiol, amine, silane, aldehyde, epoxide, isocyanate, acrylate or carboxyl group. In embodiments the PEG spacer is a Jeffamine, a PEG having an amine functional group. In additional embodiments, the PEG or PPG may be branched. For example the branched PEG or PPO may be a Y-branched or star-PEG or PPG. In embodiments these branched PEG or PPO spacers may allow multiple peptides to be conjugated to a base material through a single functional peptide.

Once a cell culture surface is formed, the spacer may act to extend the peptide (Cₐₚ) away from the cell culture surface, making the peptide more accessible to cells in culture, and improving the efficiency of the surface for cell culture. In addition, hydrophilic spacers may act to repel proteins, preventing non-specific absorption of cells or proteins to the functionalized peptide. In embodiments, the use of a cell adhesive peptide with a spacer such as PEO (polyethylene oxide) in preparing cell culture articles allows for the preparation of such articles using a lower overall concentration of adhesive peptide.

In embodiments, Sₚ may be an amino acid Xaaₙ where Xaa is independently any amino acid and n is an integer from 0 to 30, from 0 to 10, from 0 to 6 or from 0 to 3. For example, in embodiments, Sₚ may be an amino acid Xaaₙ where Xaa is G and where n = 1 to 20, or Sₚ may be an amino acid Xaaₙ where Xaa is K and n = 1 to 20, or Sₚ may be an amino acid Xaaₙ where Xaa is D and n= 1 to 20, or Sₚ may be an amino acid Xaaₙ where Xaa is E and n=1 to 20. In embodiment, spacer Sₚ may be a three amino acid sequence such as LysGlyGly or LysTyrGly. In embodiments, Xaaₙ is a series of the same amino acid. In embodiments, the spacer Sₚ may be combinations of Xaaₙ and polyethylene or polypropylene oxide. Xaaₙ may comprise a hydrophilic amino acid such as lysine, glycine, glutamic acid, aspartic acid or arginine amino acid. In embodiments, Xaaₙ may have a terminal lysine or arginine. Or, in embodiments, the spacer Sₚ may comprise polyethylene oxide spacer and amino acid spacer in any combination. In embodiments, Sₚ may be a hydrophobic spacer such as palmitic acid, stearic acid, lauric acid or hexaethylene diamine. In embodiments, Sₚ may be carboxyethyl methacrylate.

The polymerization moiety may attach to the spacer, Sₚ through the polyethylene oxide, through the side chain of an amino acid such as lysine or at the N-terminus of the amino acid. Amino acid Xaaₙ may be acetylated and/or amidated to protect it from degradation. However, if Xaaₙ is acetylated, the polymerization moiety cannot be bound to Xaaₙ through the N-terminus of the amino acid. For example, a methacrylic acid may be bound to a lysine amino acid through the side chain of the lysine amino acid where Sₚ is Xaaₙ, Xaa is lysine, n=1, and Rₘ is methacrylic acid.

In embodiments, the spacer Sₚ is Xaaₙ and Xaaₙ has a terminal lysine. In embodiments, Xaaₙ may be bound to a polymerization moiety Rₘ. For example, Xaaₙ may be (MAA)LysGlyGly or (MAA)LysTyrGly, where MAA is the polymerization moiety methacrylic acid (MAA) bound to Xaaₙ through the side chain of the terminal lysine amino acid. In additional embodiments, the polymerization moiety may be bound to the N-terminus of the Xaaₙ amino acid or amino acid chain, if the N-terminus is not acetylated. Each functionalized peptide has at least one polymerization moiety, and may have more than one. Cₐₚ is a peptide or polypeptide having a cell adhesive or cell binding sequence. In embodiments the cell adhesive or cell binding sequence is RGD.

In embodiments, the first pre-polymer may be formed from the polymerization product of functionalized peptides, as described above. In additional embodiments, the first pre-polymer may be formed from the polymerization product of functionalized peptides copolymerized in any form, for example as random copolymers or block copolymers, with additional monomers. In embodiments these additional monomers are ethylenically unsaturated monomers, including, for example, (meth)acrylate monomers include lower alkyl, i.e. C₁ to C₂₀alkyl, (meth)acrylates, e.g. methyl (meth)acrytate, ethyl (meth)acrylate, propyl (meth)acrylate, n-butyl (meth)acrylate, iso-butyl (meth)acrylate, t-butyl (meth)acrylate, 2-ethyl hexyl (meth)acrylate. octyl (meth)acrylate or dodecyl (meth)acrylate. Additionally, cyclic alkyl monomeric species may be used such as cyclohexyl (meth)acrylate, isobornyl (meth)acrylate and dicyclopentenyl (meth)acrylate. Functional monomers such as methacrylic acid and acrylic acid, hydroxy alkyl methacrylates such as hydroxy ethyl (meth)acrylate (HEMA), hydroxypropyl (meth)acrylate and hydroxybutyl (meth)acrylate, glycidyl (meth)acrylate, dialkyl aminoalkyl (meth)acrylates such as dimethyl aminoethyl (meth)acrylate, diethyl aminoethyl (meth)acrylate, dimethyl aminopropyl (meth)acrylate and diethyl aminopropyl (meth)acrylate. By (meth)acrylate, we mean that either the methacrylate or the analogous acrylate may be used. In embodiments, the additional monomers may be non-ionic. In embodiments the additional monomers may be hydrophilic.

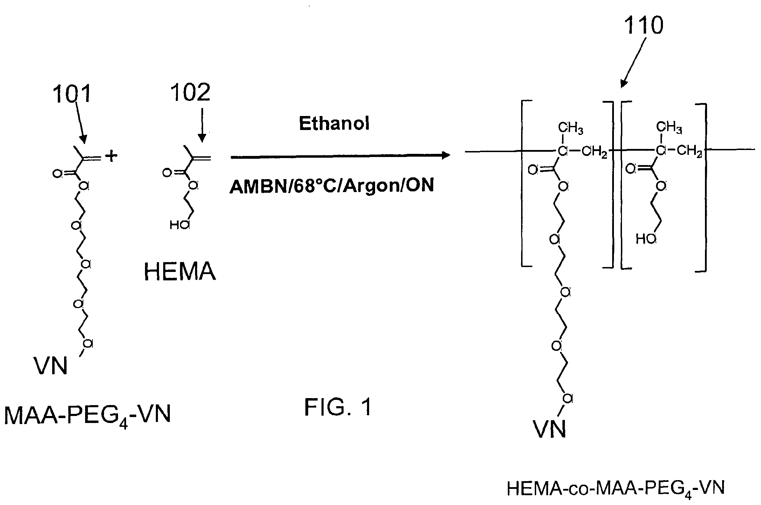

**FIG.** 1 is an illustration showing a method for making Poly(HEMA co-MAA-PEG₄-VN), an embodiment of the first pre-polymer, from a functionalized cell adhesive peptide pre-polymer. As shown in **FIG. 1****,** a functionalized peptide **101** having a PEG₄ spacer and a methacrylic acid (MAA) polymerization moiety. Referring back to Formula 1: Rₘ - Sₚ - Cₐₚ, according to the embodiment illustrated in **FIG. 1****,** Rm is MAA, Sp is PEG₄ and Cap is VN, a vitronectin sequence, which may be any of the sequences shown in Table 1 labeled VN, containing an RGD sequence. As shown in **FIG. 1****,** a MAA-PEG₄-VN functionalized peptide is reacted with an ethylenically unsaturated monomer **102,** in this case HEMA, in the presence of ethanol, a thermal initiator or a at 68° C under argon or N₂, to form an embodiment of the first pre-polymer **110,** HEMA-co-MAA-PEG₄-VN. In embodiments, the functionalized peptide pre-polymer has a Mn of >10,000 Daltons (typically 20,000 - 100,000) Daltons and a Mw > 30,000 Daltons (typically 50,000 to 300,000).

In additional embodiments, the first pre-polymer, the functionalized peptide pre-polymer is formed from a pre-polymer having a backbone formed from a plurality of ethylenically unsaturated monomers, where one of the ethylinically unsaturated monomers has a carboxyl functional group. In embodiments ethylinically unsaturated monomers which form the first pre-polymer may have carboxyl functional groups. Those of skill in the art will recognize that a carboxyl functional group may be incorporated into the ethylenically unsaturated monomers listed above, where there are suitable functional groups, without undue experimentation. An example of a monomer having a carboxyl functional group is 2-carboxyethylacrylate (CEA), although many carboxyl-group containing monomers may be provided. In embodiments, the ethylenically unsaturated monomer forming the first pre-polymer not having a carboxyl group is hydrophilic. In embodiments, the ethylenically unsaturated monomer forming the first pre-polymer having a carboxyl group is hydrophilic.

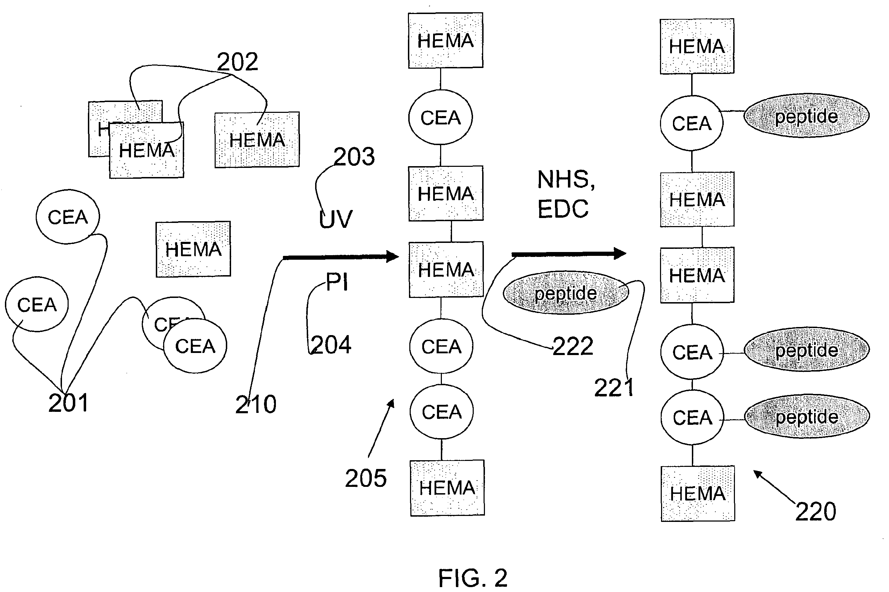

**FIG. 2** is diagram illustrating a reaction for forming an embodiment of the first pre-polymer. In **FIG. 2****,** two ethylenically unsaturated monomers, HEMA **202** and CEA **201** are provided. These two monomers are polymerized, in a reaction step **210** (by adding a polymerization initiator PI, 204) to form a HEMA-co-CEA polymer **205.** The polymerization is performed in the presence of a polymerization initiator (PI) **204** by UV radiation **203.** A peptide **221** is then bound to the HEMA-co-CEA polymer **205** to form by reaction **222** a peptide-conjugated HEMA-co-CEA polymer **220.** One suitable technique involves 1-ethyl-3-[3-dimethylaminopropyl] carbodiimide hydrochloride (EDC)/ *N*-hydroxysuccinimide (NHS) chemistry, as generally known in the art. EDC and NHS or *N*-hydroxysulfosuccinimide (sulfo-NHS) can react with carboxyl groups of the HEMA-co-CEA polymer to produce amine reactive NHS esters. In embodiments of the present invention, the incorporation of a monomer having a carboxyl group for conjugation with peptides is important. EDC reacts with a carboxyl group of the HEMA-co-CEA polymer to produce an amine-reactive *O-*acylisourea intermediate that is susceptible to hydrolysis. The addition of NHS or sulfo-NHS stabilizes the amine-reactive *O*-acylisourea intermediate by converting it to an amine reactive NHS or sulfo-NHS ester, allowing for two step procedures. Following activation HEMA-co-CEA polymer, the peptide **221** may then be added and the terminal amine of the peptide can react with the amine reactive ester to form a stable amide bond, thus conjugating the polypeptide to the HEMA-co-CEA polymer, forming the peptide-conjugated HEMA-co-CEA polymer **220.** EDC/NHS chemistry results in a zero length crosslinking of polypeptide using NHS, EDC chemistry. The excess COOH may be blocked with, for example, ethanolamine.

In embodiments, the first pre-polymer comprises the polymerization product of an ethylenically unsaturated monomer having a carboxyl group with an ethylenically unsaturated monomer not having a carboxyl group, wherein a cell adhesive peptide is conjugated to the first pre-polymer via the carboxyl group. In embodiments, the ethylenically unsaturated monomer, which does not have a carboxyl group, used to make the first pre-polymer and the ethylenically unsaturated monomer used to make the second pre-polymer are the same monomer.

In embodiments, the cell adhesive peptide or cell adhesive polypeptide (which terms are interchangeable) (Cₐₚ) has a cell binding or cell adhesive sequence which may, for example, be an integrin binding sequence or an R-G-D sequence. In embodiments, the cell adhesive peptide is a sequence of amino acids found in vitronectin, laminin, bone sialoprotein, collagen, or fibronectin. For the purposes of this disclosure, peptide or polypeptide is an amino acid sequence that may be chemically synthesized or made by recombinant methods. However, for the purposes of this disclosure, peptide or polypeptide is a fragment of a protein, and not a complete protein. In addition, peptide or polypeptide is not isolated from an animal source. In embodiments, peptide or polypeptide may include an amino acid sequence of Yaa₁ProGlnValThrArgGlyAspValPheThrMetPro (SEQ ID NO:32), a vitronectin peptide sequence where 1 is an integer from 0 to 3 and where Yaa may be any amino acid or may include, for example, lysine of which the terminal amino acid must be lysine or arginine to accommodate attachment of a polymerizable group. In embodiments , the peptide or polypeptide may be cyclic. For example RGDYK(SEQ ID NO:33) may be cyclic c(RGDyK).

Examples of peptides that may be used in embodiments are listed in Table 1.

**Table 1**

| **Sequence** | **Source** |
|---|---|
| KGGGQKCIVQTTSWSQCSKS (SEQ ID NO:1) | Cyr61 res 224-240 |
| GGGQKCIVQTTSWSQCSKS (SEQ ID NO:2) | Cyr61 res 224-240 |
| KYGLALERKDHSG (SEQ ID NO:3) | TSP1 res 87-96 |
| YGLALERKDHSG (SEQ ID NO:4) | TSP1 res 87-96 |
| KGGSINNNRWHSIYITRFGNMGS (SEQ ID NO:5) | mLMα1 res 2179-2198 |
| GGSINNNRWHSIYITRFGNMGS (SEQ ID NO:6) | mLMα1 res 2179-2198 |
| KGGTWYKIAFQRNRK (SEQ ID NO:7) | mLMα1 res 2370-2381 |
| GGTWYKIAFQRNRK (SEQ ID NO:8) | mLMα1 res 2370-2381 |
| KGGTSIKIRGTYSER (SEQ ID NO:9) | mLMγ1 res 650-261 |
| GGTSIKIRGTYSER (SEQ ID NO:10) | mLMγ1 res 650-261 |
| KYGTDIRVTLNRLNTF (SEQ ID NO:11) | mLMγ1 res 245-257 |
| YGTDIRVTLNRLNTF (SEQ ID NO:12) | mLMγ1 res 245-257 |
| KYGSETTVKYIFRLHE (SEQ ID NO:13) | mLMγ1 res 615-627 |
| YGSETTVKYIFRLHE (SEQ ID NO:14) | mLMγ1 res 615-627 |
| KYGKAFDITYVRLKF (SEQ ID NO:15) | mLMγ1 res 139-150 |
| YGKAFDITYVRLKF (SEQ ID NO:16) | mLMγ1 res 139-150 |
| KYGAASIKVAVSADR (SEQ ID NO:17) | mLMα1 res2122-2132 |
| YGAASIKVAVSADR (SEQ ID NO:18) | mLMα1 res2122-2132 |
| CGGNGEPRGDTYRAY (SEQ ID NO:19) | BSP |
| GGNGEPRGDTYRAY (SEQ ID NO:20) | BSP |
| GGNGEPRGDTRAY (SEQ ID NO:21) | BSP-Y |
| GGNGEPRGDTRAY (SEQ ID NO:22) | BSP-Y |
| KYGRKRLQVQLSIRT (SEQ ID NO:23) | mLMα1 res 2719-2730 |
| YGRKRLQVQLSIRT (SEQ ID NO:24) | mLMα1 res 2719-2730 |
| KGGRNIAEIIKDI (SEQ ID NO:25) | LMβ1 |
| GGRNIAEIIKDI (SEQ ID NO:26) | LMβ1 |
| KGGPQVTRGDVFTMP (SEQ ID NO:27) | VN |
| GGPQVTRGDVFTMP (SEQ ID NO:28) | VN |
| GRGDSPK (SEQ ID NO:29) | Short FN |
| KGGAVTGRGDSPASS (SEQ ID NO:30) | Long FN |
| GGAVTGRGDSPASS (SEQ ID NO:31) | Long FN |
| Yaa₁PQVTRGNVFTMP (SEQ ID NO:32) | VN |
| RGDYK (SEQ ID NO:33) | RGD |

It will be understood that the amount of peptide present or accessible from the cell culture surface can vary depending on the composition of the pre-polymers, the length of spacers, and the nature of the polypeptide itself. In addition, different cells in culture may respond differently to the composition of the pre-polymers as well as the identity and amount of peptide available on the surface. As discussed below in the Examples, higher densities of peptide may be better able to support attachment and proliferation of certain cell types such as, for example, undifferentiated stem cells in a chemically defined medium, although other cell types may proliferate more successfully at different peptide densities.

In embodiments, the cross-linker moiety conjugated to a second polymer backbone may be a photoreactive moiety or a thermally reactive moiety. For example, the cross-linker moiety may be an α, β unsaturated ketone photo-reactive groups. A photo-reactive group is a molecule or moiety that forms a highly reactive species upon exposure to light. Examples of photo-reactive groups include aryl azides, diazarenes, beta carbonyldiazo and benzophenones, acetophenones and derivatives thereof. Reactive species include nitrenes, carbenes and radicals. The reactive species are generally capable of forming covalent bonds. For example, in embodiments the photosensitive moiety is a benzophenone containing moiety, substituted arylazide containing moiety or trifluoromethylaryldiazirine containing moiety. In embodiments, the second pre-polymer is referred to as "Photo-HEMA." For the purposes of this disclosure, "Photo-HEMA" is a poly HEMA prepolymer having a conjugated cross-linker moiety. For example, the cross-linker moiety may be a benzophenone containing moiety.

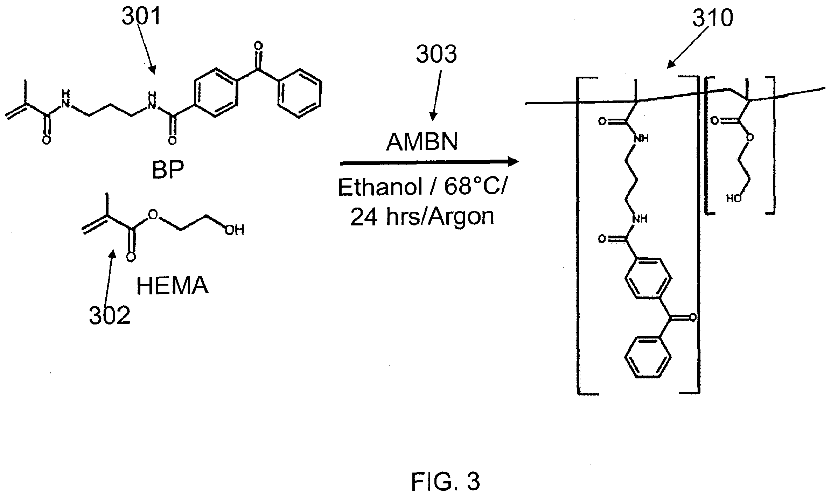

**FIG. 3** is an illustration showing a method for making an embodiment of the second pre-polymer (Photo-HEMA). **FIG. 3** shows a benzophenone monomer **301** and a HEMA monomer **302.** These monomers may be polymerized **303** in the presence of an initiator such as 2, 2'-Azobis-(2-Methylbutyronitrile) (AMBN), to form the second pre-polymer, **310.** This Photo-HEMA pre-polymer is an embodiment of a second pre-polymer wherein the second pre-polymer comprises a cross-linker moiety conjugated to a second polymer backbone. Measurements indicate that the copolymerization of N-(3-methacrylamidopropyl)-4-benzoylbenzamide and HEMA is higher than 90% (results not shown). PhotoHEMA typically has a Mn >10,000 and a Mw > 30,000.

Any suitable initiator may be used. Examples of polymerization initiators include organic peroxides, azo compounds, quinones, nitroso compounds, acyl halides, hydrazones, mercapto compounds, pyrylium compounds, imidazoles, chlorotriazines, benzoin, benzoin alkyl ethers, diketones, phenones, or mixtures thereof. Examples of suitable commercially available, ultraviolet-activated and visible light-activated photoinitiators have tradenames such as IRGACURE 651, IRGACURE 184, IRGACURE 369, IRGACURE 819, DAROCUR 4265 and DAROCUR 1173 commercially available from Ciba Specialty Chemicals, Tarrytown, N.Y. and LUCIRIN TPO and LUCIRIN TPO-L commercially available from BASF (Charlotte, N.C.)

Additional initiators may include water soluble azo-initiators that can be used in thermal polymerization including, for example, (VA-044) 2,2'-Azobis[2-(2-imidazolin-2-yl)propane]dihydrochloride; (VA046B) 2,2'-Azobis[2-(2-imidazolin-2-yl)propane]disulfate dehydrate; (VA-50) 2,2'-Azobis(2-methylpropionamidine)dihydrochloride; (VA-057) 2,2'-Azobis[N-(2-carboxyethyl)-2-methylpropionamidine]hydrate; (VA-060) 2,2'-Azobis{2-[1-(2-hydroxyethyl)-2-imidazolin-2-yl]propane}dihydrochloride; (VA-061) 2,2'-Azobis[2-(2-imidazolin-2-yl)propane]; (VA-067) 2,2'-Azobis(1-imino-1-pyrrolidino-2-ethylpropane)dihydrochloride; (VA-080) 2,2'-Azobis{2-methyl-N-[1,1-bis(hydroxymethyl)-2-hydroxyethl]propionamide or (VA-086) 2,2'-Azobis[2-methyl-N-(2-hydroxyethyl)propionamide]. Oil soluble azo-initiators such as (V-70) 2,2'-Azobis(4-methoxy-2.4-dimethyl valeronitrile); (V-65) 2,2'-Azobis(2.4-dimethyl valeronitrile); (V-601) Dimethyl 2,2'-azobis(2-methylpropionate); (V-59) 2,2'-Azobis(2-methylbutyronitrile; (V-40) 1,1'-Azobis(cyclohexane-1-carbonitrile); (VF-096) 2,2'-Azobis[N-(2-propenyl)-2-methylpropionamide]; (V-30) 1-[(1-cyano-1-methylethyl)azo]formamide; (VAm-110) 2,2'-Azobis(N-butyl-2-methylpropionamide) or (VAm-111) 2,2'-Azobis(N-cyclohexyl-2-methylpropionamide) may also be used in thermal polymerization. These initiators are available from for example, WAKO Chemicals, Richmond VA. In addition, macro-initiators, such as azo-initiators having a PEG backbone may be used in thermal polymerization.

A photosensitizer may also be included in a suitable initiator system. Representative photosensitizers have carbonyl groups or tertiary amino groups or mixtures thereof. Photosensitizers having a carbonyl groups include benzophenone, acetophenone, benzil, benzaldehyde, o-chlorobenzaldehyde, xanthone, thioxanthone, 9,10-anthraquinone, and other aromatic ketones. Photosensitizers having tertiary amines include methyldiethanolamine, ethyldiethanolamine, triethanolamine, phenylmethyl-ethanolamine, and dimethylaminoethylbenzoate. Commercially available photosensitizers include QUANTICURE ITX, QUANTICURE QTX, QUANTICURE PTX, QUANTICURE EPD from Biddle Sawyer Corp, Crawley, England.

In general, the amount of photosensitizer or photoinitiator may vary from about 0.01 to 10% by weight. Examples of cationic initiators include salts of onium cations, such as arylsulfonium salts, as well as organometallic salts such as ion arene systems.

In embodiments, the disclosure also provides methods of making a cell culture surface comprising providing the first pre-polymer with the second pre-polymer, in the presence of cell culture substrate or base, exposing the first pre-polymer and second pre-polymer mixture to an energy source to form a reactive polymer blend conjugated to the cell culture base. In additional embodiments, the disclosure provides synthetic cell culture surfaces made from the pre-polymer compositions and methods for culturing cells on the synthetic cell culture surfaces so made.

In embodiments, the disclosure provides a method for making a cell culture surface comprising mixing a first pre-polymer comprising the polymerization product of a functionalized cell adhesive peptide (**101** of FIG. 1, for example) of the formula Rₘ - Sₚ - Cₐₚ; wherein R is a polymerization moiety selected from the group consisting of acrylate , methacrylate, acrylamide, methacrylamide, maleimide or, fumarate and combinations, and m is an integer greater than 1; wherein Sₚ is an optional spacer moiety wherein the spacer moiety comprises polyethylene oxide or polypropylene oxide having the formula (O-CH₂CHR')ₘ₂ where R' is H or CH₃ and m2 is an integer from 0 to 20, or Xaaₙ wherein Xaa is independently any amino acid and n is an integer from 0 to 3, or a combination; and, wherein Cₐₚ is a peptide comprising a cell adhesive sequence with a second pre-polymer wherein the second pre-polymer comprises a cross-linking moiety conjugated to a second polymer backbone wherein the second pre-polymer comprises the polymerization product of an ethylenically unsaturated monomer and the cross-linker moiety is a photo-reactive group selected from the group consisting of aryl azides, diazarenes, beta carbonyldiazo, benzophenones, and acetophenones.

In embodiments, the disclosure provides a method for making a cell culture surface comprising mixing a first pre-polymer comprising the polymerization product of a functionalized cell adhesive peptide of the formula Rₘ - Sₚ - Cₐₚ; wherein R is a polymerization moiety selected from the group consisting of acrylate, methacrylate, acrylamide, methacrylamide, maleimide or fumarate and combinations, and m is an integer greater than 1; wherein Sₚ is an optional spacer moiety wherein the spacer moiety comprises polyethylene oxide or polypropylene oxide having the formula (O-CH₂CHR')ₘ₂ where R' is H or CH₃ and m2 is an integer from 0 to 20, or Xaaₙ wherein Xaa is independently any amino acid and n is an integer from 0 to 3, or a combination; and, wherein Cₐₚ is a peptide comprising a cell adhesive sequence, conjugated to a first polymer backbone, with a second pre-polymer wherein the second pre-polymer comprises a cross-linking moiety conjugated to a second polymer backbone wherein the second pre-polymer comprises the polymerization product of an ethylenically unsaturated monomer and the cross-linker moiety is a photo-reactive group selected from the group consisting of aryl azides, diazarenes, beta carbonyldiazo, benzophenones, and acetophenones.

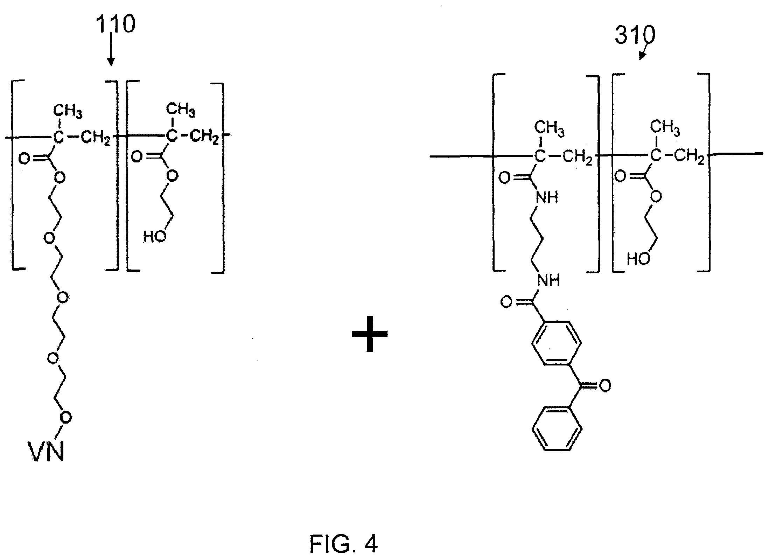

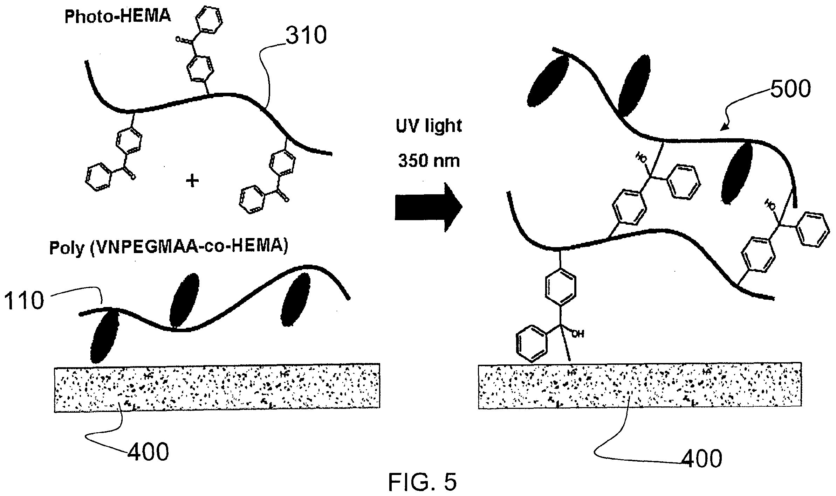

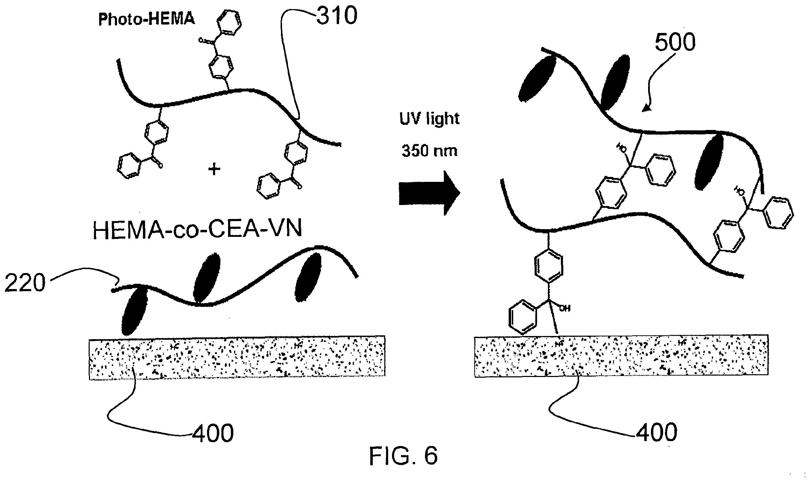

**FIG.** 4 illustrates combination of pre-polymers and Poly(HEMA co-MAA-PEG₄-VN) **110** and Photo-HEMA **310** for making an embodiment of a cell culture surface. **FIG. 5** is an illustration showing the formation of a cell culture surface by combining a first pre-polymer Poly(HEMA co-MAA-PEG₄-VN) with a second pre-polymer Photo-HEMA on a substrate **400.** Exposure of the mixture of the first pre-polymer having a peptide moiety and the second pre-polymer having photoreactive cross-linking moieties to UV radiation causes the pre-polymers to cross-link to each other and also to anchor to the substrate, forming a polymeric cell culture coating **500** on a surface **400** (see FIGs 5 and 6).

**FIG. 6** is illustration of another embodiment of a cross-linked pre-polymeric cell culture surfaces, combining Photo-HEMA **310** and Poly(HEMA co-CEA-VN) **220.** Exposure of the mixture of the first pre-polymer having a peptide moiety and the second pre-polymer having photoreactive cross-linking moieties to UV radiation causes the pre-polymers to cross-link to each other and also to anchor to the substrate, forming a polymeric cell culture coating **500** on a surface **400.**

In embodiments, the disclosure provides a method for making a cell culture article comprising providing a first pre-polymer wherein the first pre-polymer comprises a cell adhesive peptide conjugated to a first polymer backbone; and a second pre-polymer wherein the second pre-polymer comprises a cross-linker moiety conjugated to a second polymer backbone and exposing the first and second pre-polymers on the substrate to an energy source to cross-link the first and second pre-polymer to each other and to the substrate.

In embodiments, substrate may be any material suitable for culturing cells, including a ceramic substance, a glass, a plastic, a polymer or co-polymer, any combinations thereof, or a coating of one material on another. The base material may be flat or shaped. Such base materials include glass materials such as soda-lime glass, borosilicate glass, Vycor® glass or quartz glass; silicon; plastics or polymers, including dendritic polymers, such as poly(vinyl chloride), poly(vinyl alcohol), poly(methyl methacrylate), poly(vinyl acetate-*co*-maleic anhydride), poly(dimethylsiloxane) monomethacrylate, cyclic olefin polymers, fluorocarbon polymers, polystyrenes, polypropylene, polyethyleneimine; copolymers such as poly(vinyl acetate-*co*-maleic anhydride), poly(styrene-*co*-maleic anhydride), poly(ethylene-*co*-acrylic acid) or derivatives of these or the like. As used herein, "cyclic olefin copolymer" means a polymer formed from more than one monomer species, where at least one of the monomer species is a cyclic olefin monomer and at least one other monomer species is not a cyclic olefin monomer species. In many embodiments, cyclic olefin copolymers are formed from ethylene and norbonene monomers. Cyclic olefin copolymer resins are commercially available with trade name of TOPAS®Florence,KY, from Boedeker Plastics Inc, and Zeonor Corporation, Japan.

The first and the second pre-polymer may be provided to the surface of a substrate by any means known in the art including liquid dispensing, spin coating, spray coating, or other methods. Curing or polymerizing may be accomplished by any means known in the art, and depending upon the nature of the polymerizing moiety, and may include the introduction of UV, visible or thermal energy to the surface. After the cross-linking or curing step, a washing step may be desirable. Washing may be accomplished by any means known in the art including liquid dispensing and incubating, with or without agitation, where the liquid may be water, a lower alcohol, a lower alcohol diluted in water, or other solvent. In embodiments, the synthetic cell culture coating may be washed with solvent one or more times to remove impurities such as unreacted monomers or pre-polymers. In various embodiments, the layer is washed with ethanol or an ethanol-water solution, e.g. 70% ethanol, greater than 90% ethanol, greater than 95% ethanol or greater than about 99% ethanol. Washing with a 70% ethanol solvent may not only serve to remove impurities, which may be cytotoxic, but also can serve to sterilize the surface prior to incubation with cells.

In addition to the pre-polymers that form the base material layer, a composition forming the coating may include one or more additional compounds such as surfactants, wetting agents, photoinitiators, thermal initiators, catalysts and activators.

In embodiments, the cell culture surface may be formed on any surface suitable for cell culture. Examples of articles suitable for cell culture include single and multi-well plates, such as 6, 12, 96, 384, and 1536 well plates, jars, petri dishes, flasks, beakers, plates, roller bottles, slides, such as chambered and multi-chambered culture slides, tubes, cover slips, bags, membranes, hollow fibers, beads and micro-carriers, cups, spinner bottles, perfusion chambers, bioreactors, CellSTACK® (Corning, Incorporated) and fermenters

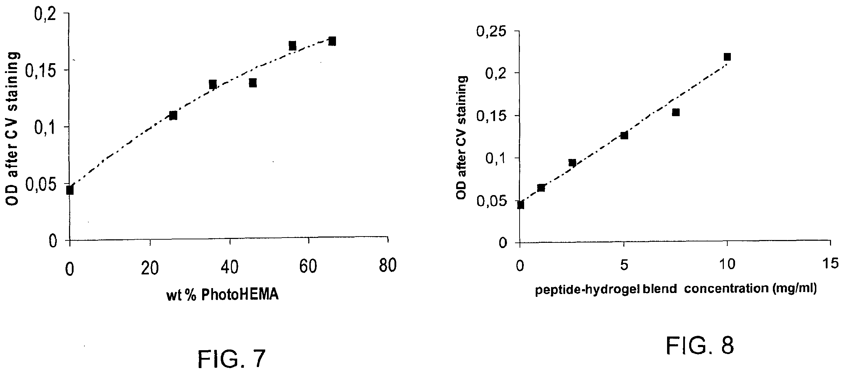

**FIG. 7** is a graph showing the amount of immobilized cell culture coating as function of the percent of the second pre-polymer (the photoreactive pre-polymer) forming copolymer in the two components mixture. The quantification is made by crystal violet staining. FIG. 7 illustrates the dose dependence of the immobilization of polymer coating as a function of the percent of the second pre-polymer present in the composition for forming a polymeric cell culture surface comprising a first pre-polymer wherein the first pre-polymer comprises a cell adhesive peptide conjugated to a first polymer backbone; and a second pre-polymer wherein the second pre-polymer comprises a cross-linker moiety conjugated to a second polymer backbone.

**FIG. 8** is a graph illustrating the amount of immobilized hydrogel as function of the concentration of the first pre-polymer, the peptide-containing pre-polymer in (mg/ml) in the solution used for coating. The quantification is made by crystal violet staining.

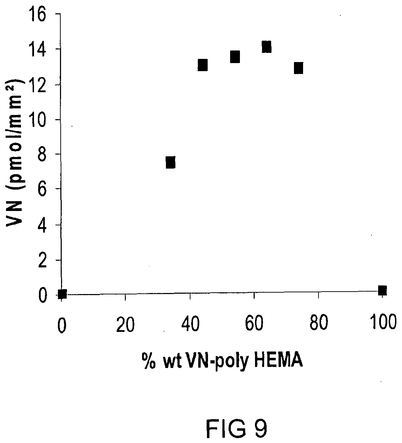

**FIG. 9** is a graph illustrating the amount of immobilized peptide (pmol/mm²) as function of the percentage by weight of the second pre-polymer, the peptide-conjugated pre-polymer in the two components mixture. The quantification is made by bicinchoninic acid (BCA) assay available from Pierce (Rockford, Ill.).

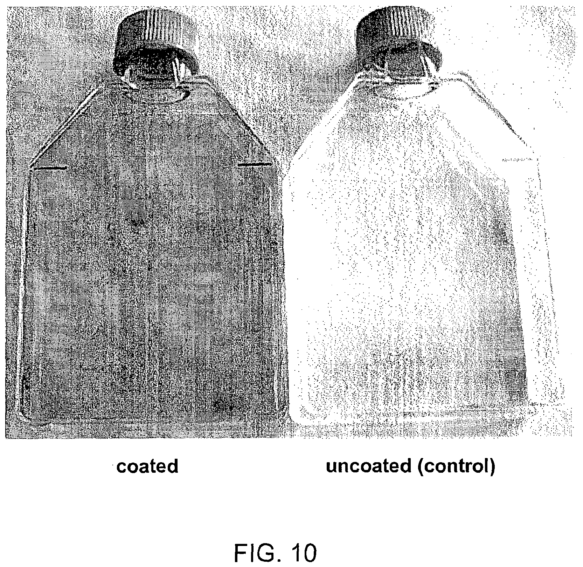

**FIG. 10** is a calibration curve allowing determination of benzophenone content of the Photo-reactive hydrogel forming copolymer after isolation and purification. The calibration curve gives the optical density (OD) at 336 nm of a solution as of N-(3-methacrylamidopropyl)-4-benzoylbenzamide (1 cm optical path) at various concentrations. Measurement of 20 mg/ml Photo-reactive hydrogel forming copolymer solution leads to OD=0.907 @ 336 nm which gives 1.38 mg/ml N-(3-methacrylamidopropyl)-4-benzoylbenzamide thus 6.9 wt% N-(3-methacrylamidopropyl)-4-benzoylbenzamide in the photo-reactive hydrogel forming copolymer (7.6 wt% expected from monomers feed value). This result indicates that the copolymerization of the N-(3-methacrylamidopropyl)-4-benzoylbenzamide and HEMA is higher than 90%.

**FIG. 10** is a photograph showing colloidal gold staining (Colloidal Gold Total Protein Stain reagent available from Bio-Rad, Hercules, CA) of a flask coated according to embodiments of the present invention and an uncoated control flask.

Cells may be used for any suitable purpose, including (i) obtaining sufficient amounts of undifferentiated stem cells cultured on a synthetic surface in a chemically defined medium for use in investigational studies or for developing therapeutic uses, (ii) for investigational studies of the cells in culture, (iii) for developing therapeutic uses, (iv) for therapeutic purposes, (v) for studying gene expression, e.g. by creating cDNA libraries, and (vi) for studying drug and toxicity screening.

Cell culture articles prepared according to embodiments of the methods of the present invention can be effectively presented on the interface of a hydrophilic surface to facilitate growth and proliferation of any relevant cell type, including, for example, cell lines such as HT1080 cells, Nulli-SCC1 cells, ES-D3 cells, stem cells, adult stem cells, Embryonic Stem Cells (ESCs) or Inducible Pluripotent cells (IPCs). These cells in culture may be used in therapeutic applications..

Other suitable stem cells include induced primate pluripotent (iPS) stem cells OPCs according to the invention may also be differentiated from induced primate pluripotent stem (iPS) cells. iPS cells refer to cells, obtained from a juvenile or adult mammal, such as a human, that are genetically modified, e.g., by transfection with one or more appropriate vectors, such that they are reprogrammed to attain the phenotype of a pluripotent stem cell such as an hESC. Phenotypic traits attained by these reprogrammed cells include morphology resembling stem cells isolated from a blastocyst as well as surface antigen expression, gene expression and telomerase activity resembling blastocyst derived embryonic stem cells. The iPS cells typically have the ability to differentiate into at least one cell type from each of the primary germ layers: ectoderm, endoderm and mesoderm and thus are suitable for differentiation into a variety of cell types. The iPS cells, like hESC, also form teratomas when injected into immuno-deficient mice, e.g., SCID mice. (Takahashi et al., (2007) Cell 131(5):861; Yu et al., (2007) Science318:5858).

Embodiments of the present invention provide for efficient techniques for decorating surfaces with cell binding adhesive ligands such as peptides in a cost effective manner and facile manufacturing processes leading to overall significant reduction in manufacturing costs. In embodiments, the surfaces are useful surfaces for culturing cells, including human embryonic stem cells in the pluripotent (having more than one potential outcome) state using chemically defined media. The use of chemically defined media, in combination with synthetic surfaces in embodiments of the present invention is important because the use of serum introduces undefined factors into cell culture which may be detrimental to cultured cells intended for therapeutic uses.

Cell culture coatings prepared using the pre-polymer methods disclosed herein have advantages over previously described methods. In particular, this method eliminates manufacturing steps compared to the in-situ polymerization of monomers so the process of the present invention is expected to be more controllable and significantly less expensive than previously disclosed processes. In embodiments, the process of coating disclosed herein allows preparation of coated article at high throughput. In addition, a very efficient crosslinking and anchoring of the cell culture coating on various non-treated substrates of interest can be achieved. So the immobilization of the cell culture coating is robust and no delamination or dissolution of the coating occurs during cell culture. The anchored cell culture coating is resistant to ethanol washing and thus the coated article can be sanitized by means of ethanol water mixture. Properties of the coating such as peptide density, crosslinking density can be finely tuned by simply altering the solid content and ratio between the two pre-polymer components of the composition. Such fine-tuning enables the preparation, from the same raw materials, of a broad range of coatings exhibiting various properties such as peptide density gel thickness, tuned crosslinking and stiffness. In term of process, the method used to crosslink and attach the polymer to the substrate is performed under air and so does not require an inert gas purge (cost saving). Light curing conditions are compatible with a standard UV Fusion conveyor system using a mercury electrodeless lamp. We have also surprisingly discovered that the composition of the invention is advantageously cross-linked and attached to the substrate by means of an UV light exposure through the polystyrene material constituting the wall, top or bottom of the article to be coated. In that case the polystyrene material plays the role of a very efficient UV filter and block short UV wavelength which have deleterious effect on adhesion peptide. This open the door to simple process allowing exposure in assembled flasks or closed vessels without the need of using top-less flasks or disassembled vessels that must be assembled further and increase the process complexity. In addition, the composition of the invention enables an unlimited shelf-life of the composition provided that the storage is made properly, protected from light, appropriate temperature and preservative added to avoid peptide degradation. Finally, coatings prepared accordingly to embodiments of the invention support stem cell attachment and growth and allows cost effective preparation of coated plasticware having adhesion peptide density of at least 10 to 15 pmol/mm², the peptide being active and available for specific cell attachment in chemically defined media.

In the following, non-limiting examples are presented, which describe various embodiments of the articles and methods discussed above.

### EXAMPLES:

### Preparation of N-(3-methacrylamidopropyl)-4-benzoylbenzamide

Step a: Synthesis of 4-benzoylbenzoyl chloride. The synthesis followed the known synthesis for benzoyl chloride¹. 4 benzoyl benzoic acid (5 g) were placed in a 100 ml round bottom flask. Thionyl chloride (15 ml) was added. The suspension was heated to reflux for one hour, during this time all solids dissolved. All volatile compounds were removed by rotary evaporation. The residue was dissolved in toluene and again evaporated. This was done two times in order to remove all thionyl chloride. The crude compound can be purified further by re-crystallization from petroleum ether (Attention the solubility is about 1 g per 100 ml). The product is a white powder, Mp. 95°C (Lit;:98-99°C). The 1H-NMR is consistent with published data. 1H NMR (400 MHz, CDCl3) δ 8.20 (d, J = 7.5, 2H), 7.86 (d, J = 7.4, 2H), 7.78 (d, J = 7.4, 2H), 7.61 (d, J = 6.7, 1H), 7.49 (t, J = 7.4, 2H) ppml3C NMR (101 MHz, CDCl3) δ 195.31, 167.85, 143.27, 136.36, 135.75, 133.34, 131.15, 130.09, 130.01, 128.59 ppm Note: Hydrolysis can be easily seen in the carbon spectra the =O signal is at 170.42 ppm.

Step b: Synthesis of N-(3-methacrylamidopropyl)-4-benzoylbenzamide: 730 mg (4.1 mmol) of N(3-aminopropyl)methacrylamide hydrochloride were solved in 25 ml dichloromethane in a 50 ml round bottom flask. 1 g (4.1 mmol) of 4 -benzoylbenzoyl chloride and phenothiazine (1 mg; as polymerization inhibitor) were added as a solid. The suspension was cooled with an ice bath while triethyl amine (1.3 ml, 10 mmol) was added. The ice bath was removed and stirred for 3 h. The organic phase was washed twice with 0.1 N HCl and dried over sodium sulfate. The solvent were evaporated under reduced pressure. The remaining crude product was purified by Flash column chromatography using ethyl acetate as solvent (Rf=0.5). The yield was 510 mg (36%) of the pure compound. ¹H NMR (400 MHz, CDCI3) δ 8.01 (d, J = 7.5, 2H), 7.86 (d, J = 7.4, 2H), 7.80 (d, J = 7.2, 2H), 7.62 (s, ¹H), 7.51 (d, J = 7.5, 2H), 5.81 (s, ¹H), 5.39 (s, ¹H), 3.49 (4H), 2.01 (s, 3H), 1.79 (s, 2H). ¹³C NMR (101 MHz, CDCI3) δ 196.05, 169.53, 166.74, 139.93, 139.52, 137.63, 137.07, 132.79, 130.13, 130.06, 128.39, 127.00, 120.29, 35.98, 29.76, 18.68.

### Preparation of the poly HEMA-co-CEA pre-polymer:

As illustrated in **FIG. 2****,** HEMA, 8.0 g (0.061 mmol), carboxyethylacrylate (CEA), 2.0 g ( 13.9 mmol) were added to 90 g ethanol in an amber flask equipped with a stir bar. Then 2, 2'-Azobis-(2-Methylbutyronitrile), 0.30 g (1.56 mmol) was added and stirred until completed dissolution. The solution was deoxygenated with an argon purge for 1 minute. The sealed flask was then heated for 24 hours at 68 °C under mixing and protected from light. After cooling to RT, the poly (HEMA-co-CEA) polymer was isolated by precipitation in butyl acetate .The white solid obtained was washed 3 X with butyl acetate and 3x with diisopropyl ether.

### Preparation of Vitronectin peptide-conjugated copolymer (VN-HEMA-CEA):

Also illustrated in **FIG. 2****,** Briefly, poly (HEMA-co-CEA) ,100 mg, was dissolved in 1.5 ml anhydrous DMF at 40°C under mixing. 1.67 ml EDC/NHS (400/200 mM) was added and stirred for 30 minutes to activate the carboxylic acid groups of the poly( HEMA-co-CEA) polymer. At the same time, 100 mg vitronectin-peptide and 150 µl x M of a solution of rhodamine-labelled peptide was dissolved in 2.6 ml borate buffer pH 9.2 and 2.90 ml ethanol. This solution was added to the NHS-activated polymer in DMF and stirred for 45 min. Then a solution consisting of 2 ml of 1M ethanolamine in borate buffer pH 9.2 and 1.4 ml ethanol was added and stirred for 30 minutes to block unreacted NHS groups. The polymer was purified by dialysis using a Cell Sup T1 regenerated cellulose tubular membrane having a nominal 3500 MWCO. The dialysis was run in a 40% ethanol /water solution. After 72 hrs, the solution was transferred in a beaker and lyophilized overnight at -89 °C under a 0.06 mbar pressure. The conjugated polymer is a pink free-flowing powder highly soluble in ethanol/water mixtures, DMSO, DMF, TFE.

### Preparation of MAA-PEG₄-peptide

The peptide (KGGPQVTRGDVFTMP SEQ ID NO:27) was synthesized and provided by American Peptide, Sunnyvale, CA by the following process. Preparation of (MAA-PEO₄-VN): MAA-PEO₄-Lys-Gly-Gly-Pro-Gln-Val-Thr-Arg-Gly-Asp-Val-Phe-Thr-Met-Pro-NH₂ (SEQ ID NO:27): The peptide was synthesized on 1mmol Fmoc-Rink Amide resin via Fmoc chemistry. Protecting groups used for amino acids are: t-Butyl group for and Asp and Thr, Trt group for Gln, Pbf for Arg, Boc for Lys. Fmoc protected amino acids were purchased from EMD Biosciences; Fmoc-PEG4-OH was purchased from Quanta Biodesign. Reagents for coupling and cleavage were purchased from Aldrich. Solvents were purchased from Fisher Scientific. The peptide chain was assembled on resin by repetitive removal of the Fmoc protecting group and coupling of protected amino acid. HBTU and HOBt were used as coupling reagents and NMM was used as base. 20% piperidine in DMF was used as de-Fmoc-reagent. Methacrylic acid (MAA) was coupled to the amino group of PEG4 after removal of the Fmoc protecting group. After the last coupling, resin was treated with TFA/TIS/H2O (95:3:2, v/v/v) for cleavage and removal of the side chain protecting groups. Crude peptide was precipitated from cold ether and collected by filtration. Yield 4.0gram (Synthesis yield 210.1%). Crude peptide was purified by reverse-phase HPLC; collected fractions with purity over 90% were pooled and lyophilized. Yield final product 1.035g (purification yield 25.9%).

The products were provided by American Peptide in ≥ 90% purity and were used without further purification. Ethanol was used as non-reactive diluents in the process and was purchased from Sigma-Aldrich.

### Preparation of functionalized cell adhesive peptide pre-polymer

As shown in **FIG. 1****,** HEMA, 60mg (0.46 mmol), Vitronectine-PEG₄-Methacrylate (nicknamed VN-PEG₄-MAA, 100 mg (0.05 mmol) were added to 7,5 ml ethanol in an amber flask equipped with a stir bar. Then 2, 2'-Azobis-(2-Methylbutyronitrile), 9mg was added and stirred until completed dissolution. The solution was deoxygenated with an argon purge for 1 minute. The sealed flask was then heated for 24 hours at 68 °C under mixing and protected from light. After cooling to RT, the poly (HEMA-co-VNPEGMAA) polymer was isolated by pouring the crude reaction medium in ethylacetate. The white solid obtained was washed 3x with diisopropyl ether and dried under vacuum. Although the coating described below have been made without any purification of the poly(HEMA-co-VN-PEG₄-MAA) polymer, unreacted peptides can be easily removed by means of well known processes such as continuous or discontinuous diafiltration process. Particularly efficient unreacted peptide removal can be performed using for example a 5,000 MWCO Corning Spin-X concentrator column.

### Preparation of Photo-reactive pre-polymer (Photo-HEMA)

Photo-HEMA was prepared accordingly to the reaction shown in **FIG.3****.** Briefly, 2-Hydroxyethylmethacrylate HEMA, 12.30 g (94 mmol), was added to 132.8 g of ethanol weighted in a amber flask equipped with a stir bar, N-(3-metacrylamidopropyl)-4-benzoylbenzamide (prepared according to the protocol described above), 1.023 g (2.9 mmol) was added and dissolved in this solution. After dissolution, 2, 2'-Azobis-(2-Methylbutyronitrile), 0.48 g (2.5 mmol) was added and stirred until the dissolution was complete. The solution was deoxygenated by means of an argon spurge for 1 minute. The sealed flask was then heated for 24 hours at 68 °C under mixing and protected from light. After cooling to RT, the polymer was isolated by precipitation in butyl acetate .The solid obtained was washed 3x with butyl acetate and 3x with diisopropyl ether. Then the polymer was filtered using a 20 µm porosity polypropylene filter and dried overnight under vacuum. All of the reaction steps are performed without direct exposure to UV light.

### Preparation of Photo-reactive pre-polymer (Photo-HEMA) having about 6mol % benzophenone content

In another example, and in accordance with **FIG. 3****,** Briefly, 2-Hydroxyethylmethacrylate HEMA, 12.30 g (94 mmol), was added to 132.8 g of ethanol weighted in a amber flask equipped with a stir bar, N-(3-methacrylamidopropyl)-4-benzoylbenzamide (prepared according to the protocol above], 2.11 g (6 mmol) was added and dissolved in this solution. After dissolution, 2, 2'-Azobis-(2-Methylbutyronitrile), 0.48 g (2.5 mmol) was added and stirred until the dissolution was complete. The solution was deoxygenated by means of an argon spurge for 1 minute. The sealed flask was then heated for 24 hours at 68 °C under mixing and protected from light. After cooling to RT, the polymer was isolated by precipitation in butyl acetate .The solid obtained was washed 3 X with butyl acetate and 3 x with diisopropyl ether. Then the polymer was filtered using a 20 µm porosity polypropylene filter and dried overnight under vacuum. All of the reaction steps are performed without direct exposure to UV light.

### 6-Well plates Coating:

Formulations were prepared by blending different ratio of component (i) and component (ii) where (i) is the cross-linker-functionalized pre-polymer and (ii) is the functionalized cell binding peptide pre-polymer and solid contents varying from 1 to 10 mg/ml in trifluoroethanol (TFE). Non-treated PS 6-WP Corning Incorporated plates were used. 26 to 31 µl/well were aliquoted (by hand using ¼-inch hole-template lid). The lid was replaced immediately hole-template lid by cover-plate. The formulation was allowed to spread to the edge with cover-plate in place (5 to 10 min. depending on RH, 8-10 g water/ air kg is preferred).Plates were allowed to dry by replacing the cover plate cover with filter paper 15 min at 40°C (or vacuum drying) UV curing was done with a "D" bulb, 80% power,0.5 belt speed, 1 pass, plate are exposed upside-down (bottom plate plays the role of UV filter protecting peptide). Plates were washed with PBS for 1 hour and dry under nitrogen flow. Optionally, the plates were washed with ethanol or ethanol 70% v/v in water (to ensure sanitization).

### 6-Well plates Coating using VNPEGMAA (pre-polymer 1) and Photo-HEMA (pre-polymer 2):

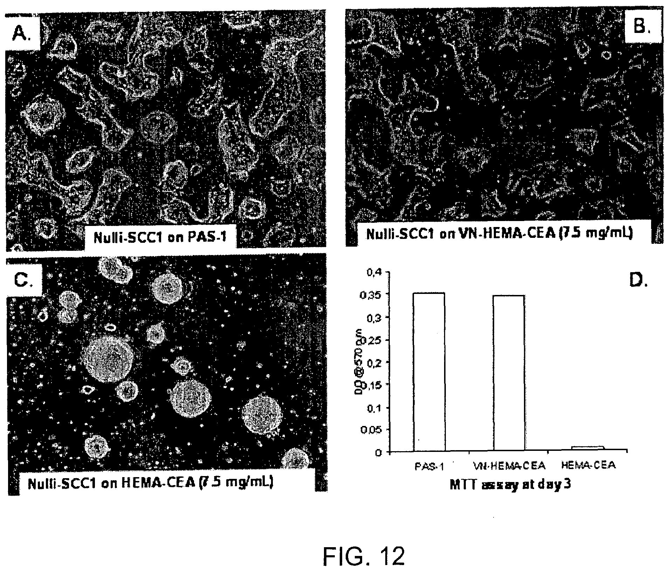

A coating formulation was prepared by blending 10 wt% Photo-HEMA (6 mol% Benzophenone as described above) as component (i-b) and 90 wt% poly (HEMA-co-VNPEGMAA) as component (ii-b) at 10 mg/ml total solid contents in trifluoroethanol (TFE) available from Sigma Aldrich. TCT (Tissue-culture-treated) PS 6-WP Corning plates were coated by dispensing 30 µl coating solution in each well. After complete solution spreading, the coating was dried for 15 min at 40°C. Then the coating was UV light-crosslinked by exposing the plate upside-down under the light provided by a Fusion "D" bulb (80% electrical power and 0.6 m/min belt speed). Then the plates were washed with 1 % aqueous sodium dodecyl sulphate for 1 hour and thoroughly rinsed with DI water and finally dried under a gentle dry gas flow. The coating was called DH2105-10 A (see Fig. 12-C for serum free ESC culture data); Immobilized peptide density determined by BCA assay shown that the immobilized peptide density is about 30-35 pmol/mm². Before cell culture, the plates were sanitized by adding ethanol 70% v/v in water or sterilized by gamma ray exposure (it is noteworthy that the present coating composition allows gamma sterilization without loss of cell adhesion performance).

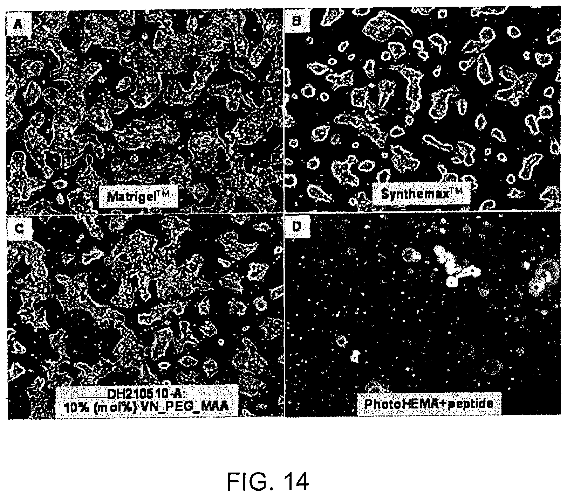

**Comparative example 1:** As a comparative example, a coating formulation was prepared by blending 10 wt% Photo-HEMA (6 mol% Benzophenone) as component (i) and 90 wt% VNPEGMAA monomer used as received from the American Peptide Company (note that in this case the acrylated-peptide was not copolymerized with HEMA) at 10 mg/ml total solid contents in trifluoroethanol. The comparative coating was nicknamed PhotoHEMA+ peptide (see **Fig. 14D**). **Fig. 14D** show clearly that when the acrylated peptide is used as a monomer, that is, without copolymerization with HEMA, the cells are not adhering to the coating. This shows that component (ii) must be a polymer and not a monomer in spite of the presence of benzophenone reactive moieties provided by component (i) which should be able to allow acrylated-peptide coupling and free radical polymerization reaction initiation.

### Cell culture:

HT1080 cells (ATCC # CCL-121) were maintained in Islove's Modified Dulbecco's Medium Life Technologies Carlsbad, CA, medium supplemented with 10% FBS and penicillin streptomycin. Cells were trypsinized and diluted before they reach confluency.

Nulli-SCCl cells (ATCC # CRL-1566) were grown on gelatine coated plates and maintained in Islove's Modified Dulbecco's Medium, Life Technologies Carlsbad, CA, medium supplemented with 10% FBS, and penicillin streptomycin. Cells were trypsinized and diluted before they reach confluency.

ES-D3 cells (ATCC # CRL-11632) were grown in Dulbecco's Modified Eagle Medium, Life Technologies Carlsbad, CA, medium supplemented with 15 % FBS and 0. ImM beta-mecaptoethanol as recommended per ATCC, Carlsbad, CA. Cells were trypsinized and diluted before they reach confluency.

**Short term adhesion assay:** Surfaces to be tested were incubated for 1 hour with 1% bovine serum albumin in D-PBS, then washed with Dulbecco's Phosphate buffered saline, Life Technologies Carlsbad, CA.

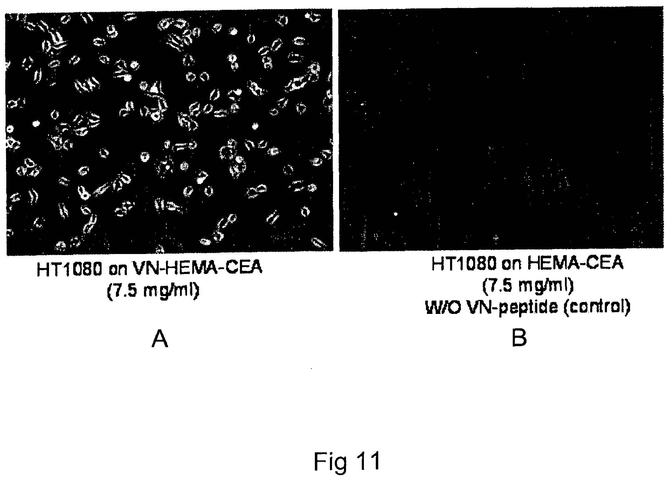

HT1080 cells were trypsinized, counted, washed in D-PBS and resuspended in DMEM without serum, supplemented with 0.1% BSA. 3.10⁵ cells per well for a 6 well plate format were seeded in 2 mL of DMEM without serum and incubated at 37°C for 1 hour. After incubation, cells were fixed 10 minutes by adjusting the medium with formaldehyde at a final concentration of 3.7%. Cells were then washed with D-PBS and representative pictures are taken. FIG. Cells are then stained with 1 mL trypan blue for 10 minutes, washed 3 times in D-PBS and lysed by addition of 200 µL of 1% SDS in water. Optic density is then measured at 570 nm on 100 µL of the lysis product. **FIG. 11A** illustrates HT1080 cells on VN-HEMA-CEA substrate. No cells are present on the HEMA-CEA substrate without peptide (**FIG. 11B**).

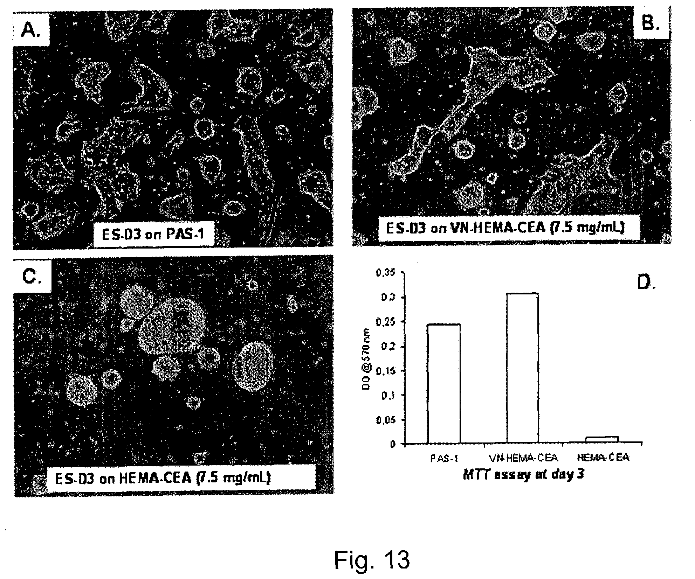

**Long term adhesion assay:** For long term adhesion assays NULLI-SCC1 or ES-D3 were collected by trypsination, counted, washed in D-PBS and resuspended in mTeSR1 synthetic medium (Stem Cell Technologies, Vancouver, BC, CA). 5.10⁵ cells per well were then seeded in 6 well plate format in 2 mL mTeSR1 and incubated at 37°C. Cell morphology was examined daily and representative pictures were taken. FIG. Medium was renewed every day. After 4 to 5 days the number of living cells was evaluated by MTT assay, briefly, cells were incubated for 1 hour with 1mL of a 5 mg/mL Thiazolyl Blue Tetrazolium Bromide solution in culture medium, then washed 2 times in D-PBS and lysed in 250 µL of lysis buffer (1% SDS, 4 mM HCl in DMSO). O.D. at 570 nm is measured on 100 µL of lysis product. **FIG. 12A** shows Nulli-SCC1 cells growing on Synthemax™ surface available from Corning Incorporated (positive control). **FIG. 12B** shows Nulli-SCCl cells on VN-HEMA-CEA surface. Cells on the VN-HEMA-CEA surface appear similar to the positive control. **FIG. 12C** shows Cells on HEMA-CEA without peptide. These cells are not attached to the cell culture surface. **FIG. 12D** shows the results of the MTT assay, showing that cells were present on the positive control (PAS-1) and on the VN-HEMA-CEA surface, but not on the negative control surface (HEMA-CEA, without peptide). Similarly, **FIG. 13A** shows ES-D3 cells growing on Synthemax™ surface available from Corning Incorporated (positive control). **FIG. 13B** shows ES-D3 cells on VN-HEMA-CEA surface. Cells on the VN-HEMA-CEA surface appear similar to the positive control. **FIG. 13C** shows ES-D3 cells on HEMA-CEA without peptide. These cells are not attached to the cell culture surface. **FIG. 13D** shows the results of the MTT assay, showing that cells were present on the positive control (PAS-1) and on the VN-HEMA-CEA surface, but not on the negative control surface (HEMA-CEA, without peptide).

**FIG. 14A-D** are photomicrographs showing ES-D3 mouse embryonic stem cell adhesion and morphology after 48 hours on Matrigel® available from BD, Franklin Lakes, NJ (**FIG. 14A**), Synthemax ™ available from Corning Incorporated, Corning, NY (**FIG. 14B**) embodiments of the polymer coating Poly(HEMA co-VN-PEG₄-MAA - 10%) (**FIG. 14C**) and a negative control of PhotoHEMA + peptide (**FIG**. **14D**) (discussed above).

Thus, embodiments of PRE-POLYMER PREPARATIONS FOR CELL CULTURE COATINGS are disclosed. One skilled in the art will appreciate that the arrays, compositions, kits articles and methods described herein can be practiced with embodiments other than those disclosed. The disclosed embodiments are presented for purposes of illustration and not limitation.

## Claims

1. A composition for forming a polymeric cell culture surface comprising:
- a first pre-polymer wherein the first pre-polymer comprises a cell adhesive peptide conjugated to a first polymer backbone; and,
- a second pre-polymer wherein the second pre-polymer comprises a cross-linker moiety conjugated to a second polymer backbone,
wherein the second pre-polymer comprises the polymerization product of an ethylenically unsaturated monomer
and the cross-linker moiety is a photo-reactive group selected from the group consisting of aryl azides, diazarenes, beta carbonyldiazo, benzophenones, and acetophenones.

2. The composition of claim 1 wherein the cell adhesive peptide comprises an R-G-D sequence.

3. The composition of claim 1 or 2 wherein the cell adhesive peptide is a sequence of amino acids found in vitronectin, laminin, bone sialoprotein, collagen, or fibronectin.

4. The composition of any one of claims 1 to 3, wherein the cell adhesive peptide is a sequence of amino acids selected from the group consisting of:
KGGGQKCIVQTTSWSQCSKS (SEQ ID NO:1),
GGGQKCIVQTTSWSQCSKS (SEQ ID NO:2), KYGLALERKDHSG (SEQ ID NO:3), YGLALERKDHSG (SEQ ID NO:4),
KGGSINNNRWHSIYITRFGNMGS (SEQ ID NO:5),
GSINNNRWHSIYITRFGNMGS (SEQ ID NO:6), KGGTWYKIAFQRNRK (SEQ ID NO:7), GGTWYKIAFQRNRK (SEQ ID NO:8), KGGTSIKIRGTYSER (SEQ ID NO:9), GGTSIKIRGTYSER (SEQ ID NO:10), KYGTDIRVTLNRLNTF (SEQ ID NO:11), YGTDIRVTLNRLNTF (SEQ ID NO:12), KYGSETTVKYIFRLHE (SEQ ID NO:13), YGSETTVKYIFRLHE (SEQ ID NO:14), KYGKAFDITYVRLKF (SEQ ID NO:15),
YGKAFDITYVRLKF (SEQ ID NO:16), KYGAASIKVAVSADR (SEQ ID NO:17), YGAASIKVAVSADR (SEQ ID NO:18), CGGNGEPRGDTYRAY (SEQ ID NO:19), GGNGEPRGDTYRAY (SEQ ID NO:20),
CGGNGEPRGDTRAY (SEQ ID NO:21), GGNGEPRGDTRAY (SEQ ID NO:22), KYGRKRLQVQLSIRT (SEQ ID NO:23), YGRKRLQVQLSIRT (SEQ ID NO:24), KGGRNIAEIIKDI (SEQ ID NO:25), GGRNIAEIIKDI (SEQ ID NO:26), KGGPQVTRGDVFTMP (SEQ ID NO:27), GGPQVTRGDVFTMP (SEQ ID NO:28), GRGDSPK (SEQ ID NO:29), KGGAVTGRGDSPASS (SEQ ID NO:30), GGAVTGRGDSPASS (SEQ ID NO:31), Yaa₁PQVTRGNVFTMP (SEQ ID NO:32), RGDYK (SEQ ID NO:33), and combinations.

5. The composition of any one of claims 1 to 4, the first pre-polymer comprising the polymerization product of a functionalized cell adhesive peptide of the formula
Rₘ - Sₚ - Cₐₚ;
wherein R is a polymerization moiety selected from the group consisting of acrylate, methacrylate, acrylamide, methacrylamide, maleimide and fumarate and any combination thereof, and m is an integer greater than 1;
wherein Sₚ is an optional spacer moiety wherein the spacer moiety comprises polyethylene oxide or polypropylene oxide having the formula (O-CH₂CHR')ₘ₂ where R' is H or CH₃ and m2 is an integer from 0 to 20, or Xaaₙ
wherein Xaa is independently any amino acid and n is an integer from 0 to 20, or any combination thereof; and,
wherein Cₐₚ is a peptide comprising a cell adhesive sequence.

6. The composition of claim 1 to 5 wherein the first pre-polymer comprises the polymerization product of an ethylenically unsaturated monomer selected from the group including (meth)acrylate monomers, methacrylic acid, acrylic acid, hydroxy alkyl methacrylates, and dialkyl aminoalkyl (meth)acrylates.

7. The composition of claim 1 to 6 wherein the first pre-polymer comprises the polymerization product of an ethylenically unsaturated monomer selected from the group including hydroxy ethyl (meth)acrylate (HEMA), hydroxypropyl (meth)acrylate, hydroxybutyl (meth)acrylate, and glycidyl (meth)acrylate.

8. The composition of any one of claims 1 to 7, wherein the first pre-polymer comprises the polymerization product of an ethylenically unsaturated monomer having a carboxyl group with an ethylenically unsaturated monomer not having a carboxyl group, wherein the cell adhesive peptide is conjugated to the first pre-polymer via the carboxyl group.

9. The composition of claim 8 wherein the ethylenically unsaturated monomer of the first pre-polymer is hydrophilic.

10. The composition of claim 8 wherein the ethylenically unsaturated monomer of the first pre-polymer not having a carboxyl group is hydrophilic.

11. The composition of claim 1 to 10 wherein the ethylenically unsaturated monomer of the second pre-polymer is hydrophilic.

12. The composition of claim 11 wherein the second pre-polymer comprises a photocrosslinkable moiety conjugated to poly-HEMA.

13. The composition of claim 12 wherein the second pre-polymer comprises a benzophenone cross-linker moiety conjugated to poly-HEMA.

14. The composition of any one of claims 9 to 13, wherein the ethylenically unsaturated monomer, which does not have a carboxyl group, used to make the first pre-polymer, and the ethylenically unsaturated monomer used to make the second pre-polymer, are the same.

15. The composition of any one of claims 1 to 14, wherein the cross-linker moiety comprises benzophenone.

16. A process of making a cell culture article comprising:
- providing the first and second pre-polymers as defined in any one of claim 1 to 15 in the presence of a substrate, and
- exposing the first and second pre-polymers on the substrate to an energy source to cross-link the first and second pre-polymer to each other and to the substrate.

17. A cell culture article obtainable by the process of claim 16.

## Patentansprüche

1. Zusammensetzung zum Bilden einer polymeren Zellkulturoberfläche, umfassend
- ein erstes Prepolymer, wobei das erste Prepolymer ein zelladhärentes Peptide umfasst, das an die Rückgratkette eines ersten Polymers konjugiert ist, und
- ein zweites Prepolymer, wobei das zweite Prepolymer einen Vernetzeranteil umfasst, der an die Rückgratkette eines zweiten Polymers konjugiert ist,
wobei das zweite Prepolymer das Polymerisationsprodukt eines ethylenisch ungesättigten Monomers umfasst
und der Vernetzeranteil eine fotoreaktive Gruppe ist ausgewählt aus der Gruppe bestehend aus Arylaziden, Diazarenen, Beta-Carbonyldiazo, Benzophenonen und Acetophenoen,

2. Zusammensetzung nach Anspruch 1, wobei das zelladhärente Peptid die R-G-D-Sequenz umfasst.

3. Zusammensetzung nach Anspruch 1 oder 2, wobei das zelladhärente Peptid eine Sequenz von Aminosäure ist, die in Vitronectin, Laminin, Knochensialoprotein, Collagen oder Fibronectin anzutreffen ist.

4. Zusammensetzung nach einem der Ansprüche 1 bis 3, wobei das zelladhärente Peptid eine Sequenz von Aminosäuren ist ausgewählt aus der Gruppe bestehend aus:
KGGGQKCIVQTTSWSQCSKS (SEQ ID NO:1), GGGQKCIVQTTSWSQCSKS (SEQ ID NO:2), KYGLALERKDHSG (SEQ ID NO:3), YGLALERKDHSG (SEQ ID NO:4),
KGGSINNNRWHSIYITRFGNMGS (SEQ ID NO:5), GSINNNRWHSIYITRFGNMGS (SEQ ID NO:6), KGGTWYKIAFQRNRK(SEQ ID NO:7), GGTWYKIAFQRNRK (SEQ ID NO:8), KGGTSIKIRGTYSER (SEQ ID NO:9), GGTSIKIRGTYSER (SEQ ID NO:10),
KYGTDIRVTLNRLNTF (SEQ ID NO:11), YGTDIRVTLNRLNTF (SEQ ID NO:12),
KYGSETTVKYIFRLHE (SEQ ID NO:13), YGSETTVKYIFRLHE (SEQ ID NO:14),
KYGKAFDITYVRLKF (SEQ ID NO:15), YGKAFDITYVRLKF (SEQ ID NO:16),
KYGAASIKVAVSADR (SEQ ID NO:17), YGAASIKVAVSADR (SEQ ID NO:18),
CGGNGEPRGDTYRAY (SEQ ID NO:19), GGNGEPRGDTYRAY (SEQ ID NO:20),
CGGNGEPRGDTRAY (SEQ ID NO:21), GGNGEPRGDTRAY (SEQ ID NO:22),
KYGRKRLQVQLSIRT (SEQ ID NO:23), YGRKRLQVQLSIRT (SEQ ID NO:24),
KGGRNIAEIIKDI (SEQ ID NO:25), GGRNIAEIIKDI (SEQ ID NO:26),
KGGPQVTRGDVFTMP (SEQ ID NO:27), GGPQVTRGDVFTMP (SEQ ID NO:28),
GRGDSPK (SEQ ID NO:29), KGGAVTGRGDSPASS (SEQ ID NO:30),
GGAVTGRGDSPASS (SEQ ID NO:31), Yaa₁PQVTRGNVFTMP (SEQ ID NO:32),
RGDYK (SEQ ID NO:33) und Kombinationen.

5. Zusammensetzung nach einem der Ansprüche 1 bis 4, wobei das erste Prepolymer das Polymerisationsprodukt eines funktionalisierten zelladhärenten Peptids der Formel
Rₘ - Sₚ - Cₐₚ
umfasst,
wobei R ein Polymerisationsanteil ist ausgewählt aus der Gruppe bestehend aus Acrylat, Methacrylat, Acrylamid, Methacrylamid, Maleimid und Fumarat und irgendeiner Kombination davon und m eine ganze Zahl über 1 ist,
wobei Sₚ ein wahlweiser Abstandhalteranteil ist, wobei der Abstandhalteranteil Polyethylenoxid oder Polypropylenoxid umfasst, das die Formel (O-CH₂CHR')ₘ₂ aufweist, wobei R' H oder CH₃ ist und m2 eine ganze Zahl von 0 bis 20 oder Xaaₙ ist,
wobei Xaa unabhängig irgendeine Aminosäure ist und n eine ganze Zahl von 0 bis 20 oder eine Kombination davon ist und
wobei Cₐₚ ein Peptid ist, das eine das zelladhärente Sequenz umfasst.

6. Zusammensetzung nach Anspruch 1 bis 5, wobei das erste Prepolymer das Polymerisationsprodukt eines ethylenisch ungesättigten Monomers umfasst ausgewählt aus der Gruppe umfassend (Meth)acrylatmonomere, Methacrylsäure, Acrylsäure, Hydroxyalkylmethacrylate und Dialkylaminoalkyl(meth)acrylate,

7. Zusammensetzung nach Anspruch 1 bis 6, wobei das erste Prepolymer das Polymerisationsprodukt eines ethylenisch ungesättigten Monomers umfasst ausgewählt aus der Gruppe umfassend Hydroxyethyl(meth)acrylat (HEMA), Hydroxypropyl(meth)acrylat, Hydroxybutyl(meth)acrylat und Glycidyl(meth)acrylat.

8. Zusammensetzung nach einem der Ansprüche 1 bis 7, wobei das erste Prepolymer das Polymerisationsprodukt eines ethylenisch ungesättigten Monomers, das eine Carboxylgruppe aufweist, mit einem ethylenisch ungesättigten Monomer, das keine Carboxylgruppe aufweist, umfasst, wobei das zelladhärente Peptid an das erste Prepolymer über die Carboxylgruppe konjugiert ist.

9. Zusammensetzung nach Anspruch 8, wobei das ethylenisch ungesättigte Monomer des ersten Prepolymers hydrophil ist.

10. Zusammensetzung nach Anspruch 8, wobei das ethylenisch ungesättigte Monomer des ersten Prepolymers, das keine Carboxylgruppe aufweist, hydrophil ist.

11. Zusammensetzung nach Anspruch 1 bis 10, wobei das ethylenisch ungesättigte Monomer des zweiten Prepolymers hydrophil ist.

12. Zusammensetzung nach Anspruch 11, wobei das zweite Prepolymer einen fotovernetzbaren Anteil umfasst, der an Poly-HEMA konjugiert ist.

13. Zusammensetzung nach Anspruch 12, wobei das zweite Prepolymer einen Benzophenon-Vernetzeranteil umfasst, der an Poly-HEMA konjugiert ist.

14. Zusammensetzung nach irgendeinem der Ansprüche 9 bis 13, wobei das ethylenisch ungesättigte Monomer, das keine Carboxylgruppe aufweist und zum Herstellen des ersten Prepolymers verwendet wird, und das ethylenisch ungesättigte Monomer, das zum Herstellen des zweiten Prepolymers verwendet wird, dieselben sind.

15. Zusammensetzung nach irgendeinem der Ansprüche 1 bis 14, wobei der Vernetzeranteil Benzophenon umfasst.

16. Verfahren zum Herstellen eines Zellkulturartikels, umfassend:
- Bereitstellen der ersten und zweiten Prepolymere, wie in irgendeinem der Ansprüche 1 bis 15 definiert, in Gegenwart eines Substrats und
- Aussetzen der ersten und zweiten Prepolymere auf dem Substrat einer Energiequelle gegenüber, um das erste und zweite Prepolymer aneinander und an das Substrat zu vernetzen.

17. Zellkulturartikel, der durch das Verfahren von Anspruch 16 erhältlich ist.

## Revendications

1. Composition pour former une surface pour culture cellulaire polymérique comprenant:
- un premier prépolymère où le premier prépolymère comprend un peptide adhésif à l'égard des cellules conjugué à un premier squelette de polymère; et
- un second prépolymère où le second prépolymère comprend une entité de réticulation conjuguée à un second squelette de polymère,
où le second prépolymère comprend le produit de polymérisation d'un monomère éthyléniquement insaturé
et l'entité de réticulation est un groupe photo-réactif choisi dans le groupe consistant en les azotures d'aryle, les diazarènes, bêta carbonyldiazo, les benzophénones et les acétophénones.

2. Composition selon la revendication 1 où le peptide adhésif à l'égard des cellules comprend une séquence R-G-D.

3. Composition selon la revendication 1 ou 2 où le peptide adhésif à l'égard des cellules est une séquence d'acides aminés trouvée dans la vitronectine, la laminine, la sialoprotéine osseuse, le collagène ou la fibronectine.

4. Composition selon l'une quelconque des revendications 1 à 3, où le peptide adhésif à l'égard des cellules est une séquence d'acides aminés choisie dans le groupe consistant en:
KGGGQKCIVQTTSWSQCSKS (SEQ ID NO:1), GGGQKCIVQTTSWSQCSKS (SEQ ID NO:2), KYGLALERKDHSG (SEQ ID NO:3), YGLALERKDHSG (SEQ ID NO:4), KGGSINNNRWHSIYITRFGNMGS (SEQ ID NO:5),
GSINNNRWHSIYITRFGNMGS (SEQ ID NO:6), KGGTWYKIAFQRNRK (SEQ ID NO:7), GGTWYKIAFQRNRK (SEQ ID NO:8), KGGTSIKIRGTYSER (SEQ ID NO:9), GGTSIKIRGTYSER (SEQ ID NO:10), KYGTDIRVTLNRLNTF (SEQ ID NO:11), YGTDIRVTLNRLNTF (SEQ ID NO:12), KYGSETTVKYIFRLHE (SEQ ID NO:13), YGSETTVKYIFRLHE (SEQ ID NO:14), KYGKAFDITYVRLKF (SEQ ID NO:15), YGKAFDITYVRLKF (SEQ ID NO:16), KYGAASIKVAVSADR (SEQ ID NO:17), YGAASIKVAVSADR (SEQ ID NO: 18),
CGGNGEPRGDTYRAY (SEQ ID NO:19), GGNGEPRGDTYRAY (SEQ ID NO:20), CGGNGEPRGDTRAY (SEQ ID NO:21), GGNGEPRGDTRAY (SEQ ID NO:22), KYGRKRLQVQLSIRT (SEQ ID NO:23), YGRKRLQVQLSIRT (SEQ ID NO:24), KGGRNIAEIIKDI (SEQ ID NO:25), GGRNIAEIIKDI (SEQ ID NO:26), KGGPQVTRGDVFTMP (SEQ ID NO:27), GGPQVTRGDVFTMP (SEQ ID NO:28), GRGDSPK (SEQ ID NO:29), KGGAVTGRGDSPASS (SEQ ID NO:30), GGAVTGRGDSPASS (SEQ ID NO:31), YaaₗPQVTRGNVFTMP (SEQ ID NO:32), RGDYK (SEQ ID NO:33), et leurs combinaisons.

5. Composition selon l'une quelconque des revendications 1 à 4, le premier prépolymère comprenant le produit de polymérisation d'un peptide adhésif à l'égard des cellules fonctionnalisé de formule Rₘ-Sₚ-Cₐₚ;
où R est une entité de polymérisation choisie dans le groupe consistant en acrylate, méthacrylate, acrylamide, méthacrylamide, maléimide et fumarate et toute combinaison de ceux-ci, et m est un entier plus grand que 1;
où Sₚ est une entité espaceur facultative où l'entité espaceur comprend un poly(oxyde d'éthylène) ou un poly(oxyde de propylène) ayant la formule (O-CH₂CHR')ₘ₂ où R' est H ou CH₃ et m2 est un entier de 0 à 20, ou Xaaₙ
où Xaa est indépendamment un acide aminé quelconque et n est un entier de 0 à 20, ou toute combinaison de ceux-ci; et
où Cₐₚ est un peptide comprenant une séquence adhésive à l'égard des cellules.

6. Composition selon les revendications 1 à 5 où le premier prépolymère comprend le produit de polymérisation d'un monomère éthyléniquement insaturé choisi dans le groupe incluant les monomères (méth)acrylates, l'acide méthacrylique, l'acide acrylique, les méthacrylates d'hydroxyalkyle et les (méth)acrylates de dialkylaminoalkyle.

7. Composition selon les revendications 1 à 6 où le premier prépolymère comprend le produit de polymérisation d'un monomère éthyléniquement insaturé choisi dans le groupe incluant le (méth)acrylate d'hydroxyéthyle (HEMA), le (méth)acrylate d'hydroxypropyle, le (méth)acrylate d'hydroxybutyle et le (méth)acrylate de glycidyle.

8. Composition selon l'une quelconque des revendications 1 à 7, où le premier prépolymère comprend le produit de polymérisation d'un monomère éthyléniquement insaturé ayant un groupe carboxyle avec un monomère éthyléniquement insaturé n'ayant pas de groupe carboxyle, où le peptide adhésif à l'égard des cellules est conjugué au premier prépolymère via le groupe carboxyle.

9. Composition selon la revendication 8 où le monomère éthyléniquement insaturé du premier prépolymère est hydrophile.

10. Composition selon la revendication 8 où le monomère éthyléniquement insaturé du premier prépolymère n'ayant pas de groupe carboxyle est hydrophile.

11. Composition selon les revendications 1 à 10 où le monomère éthyléniquement insaturé du second prépolymère est hydrophile.

12. Composition selon la revendication 11 où le second prépolymère comprend une entité photoréticulable conjuguée à poly-HEMA.

13. Composition selon la revendication 12 où le second prépolymère comprend une entité de réticulation de type benzophénone conjuguée à poly-HEMA.

14. Composition selon l'une quelconque des revendications 9 à 13, où le monomère éthyléniquement insaturé, qui n'a pas de groupe carboxyle, utilisé pour produire le premier prépolymère, et le monomère éthyléniquement insaturé utilisé pour produire le second prépolymère, sont les mêmes.

15. Composition selon l'une quelconque des revendications 1 à 14, où l'entité de réticulation comprend de la benzophénone.

16. Procédé de production d'un article pour culture cellulaire comprenant:
- l'apport des premier et second prépolymères tels que définis dans l'une quelconque des revendications 1 à 15 en présence d'un substrat, et
- l'exposition des premier et second prépolymères sur le substrat à une source d'énergie pour réticuler les premier et second prépolymères l'un à l'autre et au substrat.

17. Article pour culture cellulaire pouvant être obtenu par le procédé selon la revendication 16.
